# EUROPEAN PATENT SPECIFICATION

(11) **EP 1 434 877 B1**
(45) Date of publication and mention of the grant of the patent: **18.04.2007**
(21) Application number: 02761652.3
(22) Date of filing: 13.09.2002
(51) Int. Cl.: C12Q 1/68, C12P 19/34, C07H 21/02, A61K 49/00, A61K 39/395

(54) **CHROMOGENIC IN SITU HYBRIDIZATION METHODS, KITS, AND COMPOSITIONS**
CHROMOGENE IN-SITU-HYBRIDISIERUNGSVERFAHREN, KITS UND ZUSAMMENSETZUNGEN
PROCEDES, KITS ET COMPOSITIONS D'HYBRIDATION CHROMOGENE IN SITU

(30) Priority: 14.09.2001 US 952851; 17.06.2002 US 173525
(43) Date of publication of application: 07.07.2004
(73) Proprietor: Zymed Laboratories, Inc., South San Francisco, CA 94080 (US)
(72) Inventor: SHI, Zuo-Rong, Redwood City, CA 94061 (US); WU, Rina, San Francisco, CA 94116 (US)
(74) Representative: Harrison Goddard Foote
(86) International application number: PCT/US2002/029205
(87) International publication number: WO 2003/025127

(56) References cited:
- WO-A-00/26415
- WO-A-01/29265
- US-A- 5 643 761
- US-A- 6 070 126
- TANNER MINNA ET AL: "Chromogenic in situ hybridization: A practical alternative for fluorescence in situ hybridization to detect HER-2/neu oncogene amplification in archival breast cancer samples" AMERICAN JOURNAL OF PATHOLOGY, vol. 157, no. 5, November 2000 (2000-11), pages 1467-1472, XP002319894 ISSN: 0002-9440
- KUMAMOTO HIROYUKI ET AL: "Chromogenic in situ hybridization analysis of HER-2/neu status in breast carcinoma: Application in screening of patients for trastuzumab (Herceptin(R)) therapy" PATHOLOGY INTERNATIONAL, vol. 51, no. 8, August 2001 (2001-08), pages 579-584, XP002319895 ISSN: 1320-5463
- TANNER MINNA ET AL: "Amplification of HER-2/neu and topoisomerase IIalpha in primary and metastatic breast cancer" CANCER RESEARCH, vol. 61, no. 14, 15 July 2001 (2001-07-15), pages 5345-5348, XP002319896 ISSN: 0008-5472
- ZHAO JIANXIN ET AL: "Determination of HER2 gene amplification by chromogenic in situ hybridization (CISH) in archival breast carcinoma" MODERN PATHOLOGY, vol. 15, no. 6, June 2002 (2002-06), pages 657-665, XP002319897 ISSN: 0893-3952
- DAVISON ET AL.: 'Technical advance. Subtracted unique sequence in situ hybridization experimental and diagnostic applications' AM. J. PATHOL. vol. 153, no. 5, 1998, pages 1401 - 1409, XP002965478
- JOSSART ET AL.: 'A novel multicolor hybridization scheme applied to localization of a transcribed sequence (D10S170/H4) and deletion mapping in the thyroid cancer cell line TPC-1' CYTOGENET. CELL GENET. vol. 75, 1996, pages 254 - 257, XP002965479
- PAULETTI ET AL.: 'Assessment of methods for tissue-based detection of the Her-2/neu alteration in human breast cancer: a direct comparison of fluorescence in situ hybridization and immunohistochemistry' J. CLIN. ONCOLOGY vol. 18, 01 November 2000, pages 3651 - 3664, XP002965480
- MIYAGI ET AL.: 'Cloning and characterization of an interstitial deletion at chromosome 11p15 in a sporadic breast cancer' HUMAN MOLECULAR GENETICS vol. 1, no. 9, 1992, pages 705 - 708, XP002965481

## Description

### FIELD OF THE INVENTION

The present invention relates to chromogenic (colorimetric) *in situ* hybridization (CISH) and nucleic acid probes useful for in situ hybridization. Specifically, the present invention provides methods for performing bright-field cancer diagnostics employing chromogenic in situ hybridization (e.g. to detect gene amplifications, gene translocations, deletion, and chromosome aneuploidy). In preferred embodiments, the present invention provides CISH methods for detecting HER2 (*erb*B-2) gene status.

### BACKGROUND OF THE INVENTION

Characterization chromosome aberrations have been studied in a wide range of tumors. Specific oncogene and tumor suppressor gene targets affected by these chromosomal abnormalities have been characterized in many tumors. One such target is the HER2 gene. HER2 gene amplification or HER2 protein overexpression has been identified in 10-34% of invasive breast cancers according to a series of 52 published studies including more than 16,000 patients and using different methodologies (See, Ross et al., Am. J. Clin. Pathol., 1999; 112:S53-67, herein incorporated by reference).

Identification of HER2 status is important for determining the prognosis of patients who have invasive breast cancer, as well as for selecting a subgroup with metastasis HER2 overexpression for therapy with trastuzumab (HERCEPTIN^{®}), a humanized anti-HER2 monoclonal antibody (See, Shak et al., Cancer Res. 199; 6:71-7; and Cobleigh et al., J. Clin. Oncol., 1999; 17:2639-48,). HERCEPTIN^{®} has been found to be effective only in patients whose tumors show HER2 gene amplification and/or HER protein overexpression. As such, accurate, consistent, and straightforward methods for evaluation of HER2 status have become increasingly important.

Immunohistochemical (IHC) staining has been the predominant method of determining HER2 status in breast cancer specimens. It is relatively easy to perform and has a rapid turnaround time, and a relatively low cost (See, Ross et al. above, and Hanna et al., Mod. Pathol., 1999, 12:827-34, herein incorporated by reference). However, many commercially available antibodies have demonstrated wide variation in sensitivity and specificity for FFPE (formalin fixed paraffin embedded) tissue samples, and the effect of the tissue fixative and pretreament have a substantial effect on HER2 IHC staining (See, Ross et al. above; Jacobs et al., J. Clin. Oncol. 1999, 17:1974-1987; Espinoza et al., J. Clin. Oncol. 1999,17:2293B; and Penault-Llorca et al., J. Pathol. 1994, 173:65-75).

In addition, the lack of a universal scoring system and interobserver differences in interpretation of HER2 IHC results is also source of unwanted variation.

Overexpresion of the HER2 protein generally (>95%) results from HER2 gene amplification (See, Slamon et al., Science, 1989; 244:707-12, herein incorporated by reference). Fluorescence *in situ* hybridization (FISH) is believed by many to be the most sensitive technique for quantitative evaluation of HER2 gene status in breast cancer cells and also believed to be a valid alternative to IHC in FFPE tissue sections (See, Pauletti et al., J. Clin. Oncology, 2000, 18:3651-64). Patients who were positive by FISH but negative by IHC had a worse survival rate than those who had HER2 overexpression but an absence of gene amplification (See, Pauletti et al., above). Therefore, HER2 amplification could provide more meaningful prognostic information than HER2 overexpression in breast cancer patients. In addition, FISH quantifies the number of gene copies in the cancer cell, which objectively reflects the HER2 gene status of tumors, whereas IHC is a more subjective test. Therefore, FISH can be easier to interpret than IHC. However, FISH methodology also has many disadvantages.

Evaluation of FISH requires a modem and expensive fluorescence microscope equipped with high-quality 60X or 100X oil immersion objectives and multi-band-pass fluorescence filters, which is not used in most routine diagnostic laboratories. The fluorescence signals can fade within several weeks, and the hybridization results are typically recorded with an expensive CCD camera. Therefore, analysis and recording of FISH data is expensive and time consuming. Most importantly, tissue section morphology is not optimal in FISH on FFPE, a particular problem for distinguishing invasive breast cancer and breast carcinoma *in situ,* where HER2 gene amplification or protein overexpression may have different clinical significance. All of these limitations make FFPE FISH cumbersome for routine work (See, Jacobs et al. above, and Tanner et al., Am. J. Pathol. 2000, 157:1467-72).

Therefore, what is needed are methods that accurately identify cancer marker gene status, such as HER2 gene status, that do not require expensive fluorescence detection equipment, allow cell morphology and ISH signal to be viewed at the same time, and provide accurate results using standard equipment, such as bright field-microscopes.

A Methods for determining HER-2/neu oncogene status in breast cancer patients using chromogenic *in situ* hybridation are disclosed in Tanner *et al,* 2000 (American Journal of Pathology, vol 57 (5): I467-1472) and in Kumamoto *et al,* 2001 (Pathology International, 51:579-584), thereby enabling selection of suitable patients for trasruzumab (HERCEPTIN®) therapy. A fluorescent hybridization method applied to two targets simultaneously (HER-2/neu and Topo-II-alpha) is described in Tanner *et al,* 2001 (Cancer Research 61:5345-5348). A comparison of the efficacy of CISH with FISH in determination of the HER2 status of human breast cancer is outlined in Zhao et al, 2002 (Mod. Pathol, 1 S(6):657-665). WO 00/26415 discloses methods for producing a genomic subtractive library. WO O1/29265 discloses methods for detecting single gene copies *in*-*situ.*

### SUMMARY OF THE INVENTION

The present invention relates to chromogenic (colorimetric) *in situ* hybridization (CISH) and nucleic acid probes useful for *in situ* hybridization. Specifically, the present invention provides methods for performing bright-field cancer diagnostics employing chromogenic *in situ* hybridization (e.g. to detect gene amplifications, gene translocations, and chromosome polysomy). In preferred embodiments, the present invention provides CISH methods for detecting HER2 gene status.

In one aspect, the present invention provides methods for performing chromogenic *in-situ* hybridization, comprising: a) preheating a biological sample (e.g. tumor biopsy) in a pretreatment buffer at a temperature of at least 96 degrees Celsius, b) exposing the biological sample to a enzyme digestion solution, c) contacting the biological sample with a subtracted probe library under conditions such that the subtracted probe library hybridizes to a target region in the biological sample, d) adding a detection molecule linked to an enzyme to the biological sample under conditions such that the detection molecule binds: i) to the labeled subtracted probe library, or ii) an intermediate molecule linked to the subtracted probe library, and e) adding a colorimetric substrate to the biological sample. In embodiments, the method further comprises step f) detecting the presence or absence of the target region in the biological sample. In additional embodiments, the detecting comprising visualizing the colorimetric substrate with a microscope (e.g. bright-field microscope).

In some embodiments, the subtracted probe library is configured for detecting HER2 gene amplification. In particular embodiments, the target region comprises the HER2 gene. In other embodiments, the subtracted probe library is configured for detecting topoIIα gene amplification. In certain embodiments, the target region comprises the topoIIα gene (e.g. and does not encompass the HER2 gene sequence). In some embodiments, the subtracted probe library is configured for detecting EGFR (epidermal growth factor receptor) gene amplification. In particular embodiments, the target region comprises the EGFR gene. In other embodiments, the subtracted probe library is configured for detecting N-MYC gene amplification. In additional embodiments, the target region comprises the N-MYC gene.

In some embodiments, the subtracted probe library comprises a probe pair library. In other embodiments, the probe pair comprises a split-apart probe pair. In particular embodiments, the probe pair library comprises; i) a first probe library configured to hybridize to a first region of chromosome nine that is centromeric with respect to the ABL gene, and ii) a second probe library configured to hybridize to a second region of chromosome nine that is teleomeric with respect to the ABL gene. In other embodiments, the probe pair library comprises; i) a first probe library configured to hybridize to a first region of chromosome eighteen that is centromeric with respect to the SYT gene, and ii) a second probe library configured to hybridize to a second region of chromosome eighteen that is teleomeric with respect to the SYT gene.

In certain embodiments, the preheat temperature is at least 98 degrees Celsius (e.g. 98, 99 or 100 degrees Celsius). In other embodiments, the preheat temperature is from 96 degrees Celsius to 100 degrees Celsius (e.g. 98-100 degrees Celsius). In some embodiments, the preheating is accomplished with a pressure cooker, a hot plate, or a microwave oven. In other embodiments, the biological sample, during the preheating step, is inside an enclosed container.

In some embodiments, the enzyme digestion solution comprises pepsin (e.g., a solution having about 0.0625% pepsin, pH 2.3). In other embodiments, the pretreatment buffer comprises TRIS-EDTA (e.g. 0.1 M Tris/0.05 EDTA, pH 7.0). In other embodiments, the pretreament buffer is TRIS.

In certain embodiments, the detection molecule is avidin, streptavidin or biotin. In some embodiments, the detection molecule is an antibody. In particular embodiments, the detection molecule is linked to a plurality of enzymes via a polymer. In additional embodiments, the intermediate molecule is a primary antibody, and the detection molecule is a secondary antibody that binds to the primary antibody.

In some embodiments, the enzyme comprises a peroxidase (e.g. a horseradish peroxidase). In other embodiments, the enzyme is HRP or AP. In other embodiments, the method further comprises performing immunohistochemistry on the biological sample with antibodies specific for proteins expressed by the target region. In some embodiments, the subtracted probe library comprises digoxigenin, FITC, avidin, streptavidin, or biotin. In additional embodiments, the colorimetric substrate comprises diaminobenzidine or FAST RED.

In certain embodiments, the subtracted probe library comprises a heterogeneous mixture of labeled nucleic acid probes about 0.1 kb to about 8 kb in length (e.g. about 0.5 to about 4 kb in length). In some embodiments, the target region is about 50 kb to about 500 kb, or 1.5 to 5.0 megabases in length. In other embodiments, the target region is associated with human cancer gene aberrations. In certain embodiments, the biological sample is a tumor sample (e.g. a breast cancer biopsy tissue sample). In some embodiments, the biological sample is fixed on a surface (e.g. microscope slide).

In some embodiments, the subtracted probe library is about 90 percent free of repeat sequences. In other embodiments, the subtracted probe library is about 95 percent free of repeat sequences. In certain embodiments, the biological sample is a paraffin-embedded tissue sample (e.g. formalin-fixed paraffin-embedded tissue sample).

In another aspect, the present invention provides methods for diagnosing and treating a subject, comprising: a) preheating a biological sample from a subject in a pretreatment buffer at a temperature of at least 96°C, b) exposing the biological sample to an enzyme digestion solution, c) contacting the biological sample with a subtracted probe library under conditions such that the subtracted probe library hybridizes to a target region in the biological sample, wherein the target region comprises the HER2 gene sequence, d) adding a detection molecule linked to an enzyme to the biological sample under conditions such that the detection molecule binds: i) to the labeled subtracted probe library, or ii) an intermediate molecule linked to the subtracted probe library, e) adding a colorimetric substrate to the biological sample, f) detecting the target region by visualizing the colorimetric substrate with a bright-field microscope, thereby determining that the biological sample has amplification of the HER2 gene sequence, and g) identifying the subject as suitable for treatment with anti-HER2 antibodies. In particular embodiments, the method further comprises step h) administering the anti-HER2 antibodies (e.g. HERCEPTIN^{®}) may be administered to the subject.

In some particularly preferred embodiments, the candidate subject is a human. In other embodiments, the candidate subject is a non-human animal. In some embodiments, the animal is a mammal (*e.g.,* human, cat, dog, pig, or cow). In some preferred embodiments, the animal is a female, in other embodiments, the animal is a male. In some embodiments, the candidate subject has breast cancer cells (*e.g*., previously diagnosed as having breast cancer cells). In some preferred embodiments, the breast cancer cells are metastatic.

### DESCRIPTION OF THE FIGURES

Figure 1 shows the 3' end of the Exemplary topoIIα probe (SEQ ID NO:9), and the 5' end of the Exemplary topoIIα probe (SEQ ID NO: 10).
Figure 2 shows chart useful for interpreting ISH results using topoIIα, and chromosome 17 probes.
Figure 3 shows the BAC,clones used in Example 6 that flank the ABL gene.
Figure 4 shows ABL translocations, partner genes involved and Leukemias with ABL translocations.
Figure 5A shows a schematic diagram of ABL DNA, and Figure 5B shows various breakpoints in the ABL gene.
Figure 6 shows BCR-ABL translocations.
Figure 7 shows simplified scheme of the BCR and ABL genes with indicated breakpoints, along with exemplary BCR/ABL transcripts and proteins originating from individual breaks on the BCR and ABL genes.
Figure 8 shows clinicopathogic correlates of the most common BCR-ABL fusions.
Figure 9 shows UCSC genome browser for ABL gene.

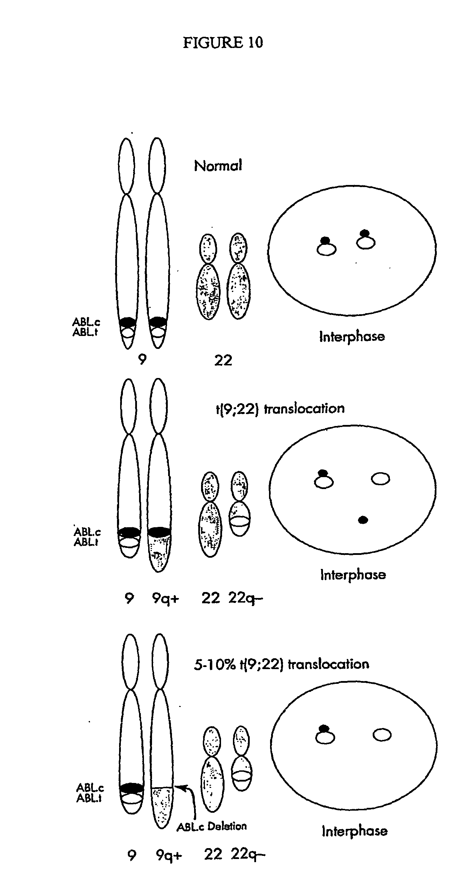

Figure 10 shows a schematic illustration of ABL translocation detection by dual-color in situ hybridization (e.g. CISH or FISH). Black dots represent ABL.c and white dots represent ABL.t. Partial karyotyptes and the corresponding interphase nuclei are shown in the figure. Normal cells without ABL translocations show black and white dots in juxtaposition, while cells with ABL translocation show one pair of black and white dots separated. Cells with ABL translocation and deletion of chromosomal material centromeric to the ABL gene breakpoint show one pair of black and white dots in juxtaposition and the black dot in another pair is disappeared (deleted).

### DEFINITIONS

To facilitate an understanding of the present invention, a number of terms and phrases are defined below:

As used herein, the term "candidate subject", "subject" or "patient" refers to an animal like a dog, cat, bird, livestock, and preferably a human. In some embodiments, the subject is suspected of having cancer that may be evaluated for suitability for topoisomerase 11 inhibitor treatment or anti-HER2 immunotherapy. Examples of subject and candidate subjects include, but are not limited to, human women suspected of having breast cancer and human men suspected of having breast cancer.

As used herein, the term "copy number" as used in reference to specific nucleic acid sequences (*e.g.,* HER-2/*neu*, *topoIIα* and control) refers to the actual number of these sequences per single cell. Copy number may be reported for one single cell, or reported as the average number in a group of cells (*e.g.*, tissue sample). When comparing the "copy number" of cells (*e.g.*, experimental and control cells) one need not determine the exact copy number of the cell, but instead need only obtain an approximation that allows one to determine whether a given cell contains more or less of the nucleic acid sequence as compared to another cell. Thus, any method capable of reliably directly or indirectly determining amounts of nucleic acid may be used as a measure of copy number even if the actual copy number is not determined.

As used herein, the term "HER-2/*neu*" refers to a nucleic acid sequence encoding the HER2 protein, and includes both the wild-type sequence and naturally occurring variations, truncations, and mutations.

As used herein, the term *"topoIIα"* refers to a nucleic acid sequence encoding TopoIIα protein, or portions thereof, and includes both the wild-type sequence and naturally occurring variations, truncations, and mutations.

As used herein, the term "suitable for treatment with topoisomerase II inhibitors" when used in reference to a candidate subject refers to subjects who are more likely to benefit from treatment with topoisomersase II inhibitors than a subject selected randomly from the population. For example, using the screening methods of the present invention as described in Example 6,79% of the subjects selected responded to topoisomerase II inhibitor treatment (as compared to 10% or less if subjects were randomly selected from the population, or as compared to approximately 30-40% of metastatic breast cancer patients).

As used herein, the term "amplification" when used in reference to copy number refers to the condition in which the copy number of a nucleic acid sequence (*e.g.,* HER-2/*neu*) is greater than the copy number of a control sequence (*e.g.,* chromosome 17). In other words, amplification indicates that the ratio of a particular nucleic acid sequence *(e.g.,* HER-2/*neu*) is greater than 1:1 when compared to a control sequence (*e.g.,* 1.1:1, 1.2:1, or 1.3:1). In preferred embodiments, the ratio of a particular nucleic acid sequence is at least 1.5 times greater than the control sequence copy number (*i.e.,* 1.5:1).

As used herein, the term "nucleic acid molecule" and "nucleic acid sequence" refer to any nucleic acid containing molecule including, but not limited to DNA or RNA. The term encompasses sequences that include any of the known base analogs of DNA and RNA including, but not limited to, 4-acetylcytosine, 8-hydroxy N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As used herein, the term "hybridization" is used in reference to the pairing of complementary nucleic acids. Hybridization and the strength of hybridization (*i.e*., the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "probe" refers to an oligonucleotide (*i.e.,* a sequence of nucleotides), or a library of nucleotide fragments, whether occurring naturally as in a purified restriction digest or produced synthetically, recombinantly or by amplification (e.g. PCR), which is capable of hybridizing to an oligonucleotide of interest. Probes useful in the present invention may be single-stranded or double-stranded. Probes are useful in the detection, identification and isolation of particular gene sequences (*e.g*., HER 2/*neu, topoIIα,* and chromosome 17). It is contemplated that any probe used in the present invention may be labeled with any "reporter molecule," so that is detectable in any detection system, including, but not limited to enzyme (*e.g*., ELISA, as well as enzyme-based immuno-histochemical assays), fluorescent (*e.g*., FISH), radioactive, mass spectroscopy, and luminescent systems. It is not intended that the present invention be limited to any particular detection system or label.

As used herein, the term "label" refers to any molecule which may be detected. For example, labels include, but are not limited to, ³²P, ¹⁴C, ¹²⁵I, ³H, ³⁵S, biotin, digoxigenin, avidin, fluorescent or enzymatic molecules.

As used herein, the phrase "repetitive nucleic acid sequences" refers to nucleic acid sequence within a genome which encompass a series of nucleotides which are repeated many times, often in tandem arrays. The repetitive sequences can occur in the genome in multiple copies ranging from two to hundreds of thousands of copies and may be clustered or interspersed on one or more chromosomes throughout a genome. Although repetitive nucleic acid sequences may be present throughout the genome, a large number of the repetitive nucleic acid sequences are typically located at the centromere of each chromosome. Examples of repetitive nucleic acid sequences include, but are not limited to, ALU and LINE elements.

As used herein, the terms *"in situ* hybridization" and "ISH" refer to methods for detecting and localizing nucleic acids within a cell or tissue preparation. These methods provide both quantitative and spatial information concerning the nucleic acid sequences within an individual cell or chromosome. ISH has been commonly used in many areas, including prenatal genetic disorder diagnosis, molecular cytogenetics, to detect gene expression and overexpression, to identify sites of gene expression, to map genes, to localize target genes and to identify various viral and microbial infections, tumor diagnosis, *in vitro* fertilization analysis, analysis of bone marrow transplantation and chromosome analysis. The technique generally involves the use of labeled nucleic acid probes which are hybridized to a chromosome or mRNA in cells that are mounted on a surface (e.g slides or other material). The probes can be labeled with fluorescent molecules or other labels. One example of fluorescent *in situ* hybridization (FISH) is provided in Kuo *et al., Am. J. Hum. Genet.,* 49:112-119, 1991. Other ISH and FISH detection methods are provided in U.S. Pat., 5,750,340 to Kim *et al.,* hereby incorporated by reference. Further examples of fluorescent *in situ* hybridization, as well as chromogenic in *situ* hybridization are provided in Examples 1-10 below. Additional protocols are known to those of skill in the art.

As used herein, the phrase "under *in situ* hybridization conditions" refers to any set of conditions used for performing *in situ* hybridization (ISH) that allows the successful detection of labeled oligonucleotide probes. Generally, the conditions used for in *situ* hybridization involve the fixation of tissue or other biological sample onto a surface, prehybridization treatment to increase the accessibility of target nucleic acid sequences in the sample (and to reduce non-specific binding), hybridization of the labeled nucleic acid probes to the target nucleic acid, post-hybridization washes to remove unbound probe, and detection of the hybridized probes. Each of these steps is well known in the art and has been performed under many different experimental conditions. Again, examples of such *in situ* hybridization conditions are provided in Kuo *et al.,* U.S. Pat. 5,750,340, and Examples 1-10 (below). Further examples of conditions and reagents useful for performing *in situ* hybridization are provided below.

The tissue or biological sample can be fixed to a surface using fixatives. Preferred fixatives cause fixation of the cellular constituents through a precipitating action which is reversible, maintains a cellular morphology with the nucleic acid in the appropriate cellular location, and does not interfere with nucleic acid hybridization. Examples of fixatives include, but are not limited to, formaldehyde, alcohols, salt solutions, mercuric chloride, sodium chloride, sodium sulfate, potassium dichromate, potassium phosphate, ammonium bromide, calcium chloride, sodium acetate, lithium chloride, cesium acetate, calcium or magnesium acetate, potassium nitrate, potassium dichromate, sodium chromate, potassium iodide, sodium iodate, sodium thiosulfate, picric acid, acetic acid, sodium hydroxide, acetones, chloroform glycerin, and thymol.

After being fixed on a surface, the samples are treated to remove proteins and other cellular material which may cause nonspecific background binding. Agents which remove protein include, but are not limited to, enzymes such as pronase and proteinase K, or mild acids, such as 0.02.-0.2 HC1, as well as RNase (to remove RNA).

DNA on the surface may then denatured so that the oligonucleotide probes can bind to give a signal. Denaturation can be accomplished, for example, by varying the pH, increasing temperature, or with organic solvents such as formamide. The labeled probe may then hybridize with the denatured DNA under standard hybridization conditions. The tissue or biological sample may be deposited on a solid surface using standard techniques such as sectioning of tissues or smearing or cytocentrifugation of single cell suspensions. Examples of solid surfaces include, but are not limited to, glass, nitrocellulose, adhesive tape, nylon, or GENE SCREEN PLUS.

As used herein, the term "polymerase chain reaction" ("PCR") refers to the method described in U.S. Patent Nos. 4,683,195, 4,683,202, and 4,965,188, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e.,* denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (*e.g.,* hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the phrase "anti-HER2 antibody-free topoisomerase II inhibitor treatment" refers to a treatment regimen for a subject that includes administering topoisomerase II inhibitors (e.g. anthracyclines), but does not include anti-HER2 antibody administration at about the same time.

As used herein, the phrase "topoisomerase II inhibitor-free anti-HER2 antibody treatment" refers to a treatment regimen for a subject that includes the administration of anti-HER2 antibodies (e.g. HERCEPTIN^{®}), but does not include topoisomerase II inhibitor (e.g. anthracyclines) administration at about the same time.

As used herein, the phrase "subtracted probe library" refers to a mixture of nucleic acid fragments configured to hybridize to a target region (e.g. selected portion of a chromosome containing gene of interest) that comprises at least about 90 percent repeat free fragments.

### DESCRIPTION OF THE INVENTION

The present invention relates to chromogenic (colorimetric) *in situ* hybridization (CISH) and nucleic acid probes useful for in situ hybridization. Specifically, the present invention provides methods, kits, and compositions for performing bright-field cancer diagnostics employing chromogenic in situ hybridization (e.g. to detect gene amplifications, gene translocations, and chromosome polysomy). In preferred embodiments, the present invention provides CISH methods, kits and compositions for detecting HER2 gene status. The description of the invention is presented below in the following sections: I. Chromogenic In-Situ Hybridization; IL CISH HER-2/neu Detection and Anti-HER2 Antibody Therapy; III. Combined HER2/*HER-2*/*neu* and *topoIIα* detection; IV. Combined CISH and IHC; V. Subtracted Probes; and VI. ABL Probe Pairs and Detecting BCR-ABL Translocations.

### I. Chromogenic In Situ Hybridization

Chromogenic in situ hybridization (CISH) is a technique that allows in situ hybridization methods to be performed and detected with a bright-field microscope, instead of a fluorescence microscope as required for FISH. While FISH requires a modem and expensive fluorescence microscopes equipped with high-quality 60X or 100X oil immersion objectives and multi-band-pass fluorescence filters (not used in most routine diagnostic laboratories), CISH allows detection with standard light (bright-field) microscopes (which are generally used in diagnostic laboratories). Also, with FISH, the fluorescence signals can fade within several weeks, and the hybridization results are typically recorded with an expensive CCD camera, while the results of CISH do not generally fade allowing the tissue samples to be archived and reviewed later. Therefore, analysis and recording of FISH data is expensive and time consuming. Most importantly, tissue section morphology is not optimal in FISH on FFPE. Generally, histological detail is better appreciated with bright-field detection, which is possible with CISH detection. A further advantage of CISH is that large regions of tissue section can be scanned rapidly after CISH counterstaining since morphological detail is readily apparent using low power objectives (e.g. 10X and 20X), while FISH detection generally requires substantially higher magnification (thus reducing the field of view). These advantages generally make CISH a superior in situ hybridization technique compared to FISH.

General chromogenic/colorimetric in situ hybridization methods are described in WO0026415 to Fletcher et al.. Particular reagents and steps for performing CISH on formalin-fixed, paraffin-embedded (FFPE) tissue samples, as well as cell sample/metaphase chromosome samples are described in WO0026415 and the section presented below. Importantly the description detailed below provides exemplary CISH methods, procedures, and reagents, and is not to be construed as limiting the present invention.

### A. Formalin-Fixed, Paraffin-Embedded (FFPE) Tissue Samples

Generally, FFPE tissue samples (e.g. cancer biopsy tissue samples) will measure about 1-2 cm in diameter, but any type of diameter maybe employed. This tissue sections (e.g. 4-5 um) may be mounted on treated (e.g. HISTOGRIP treated) microscope slides or other solid support surface (e.g. Superfrost/Plus microscope slides).

### i. PRETREATMENT

In preferred embodiments, the FFPE tissue samples are first subjected to a deparaffinization step. This may be accomplished, for example, by exposing the sample to Xylene for about 10 minutes at room temperature. This may be repeated if necessary. The sample may then be exposed to EtOH (e.g. 100% EtOH) for about 5 minutes at room temperature. In preferred embodiments, this is performed three times. The tissue samples are then allowed to dry (e.g. air dry).

Next, tissue samples are subj ected to a heat pretreament step. Specifically, a pretreatment buffer is added to the tissue samples, and the samples are heated to approximately 96-100 degrees Celsius for approximately 15 minutes (although varying incubation times may be used depending on the tissue fixation). Examples of pretreatment buffers included, but are not limited to, Citrate buffer, EDTA-TRIS buffer (e.g. 0.1M Tris/0.05 M EDTA, pH 7.0), and TRIS buffer. In certain embodiments, the preheat temperature is achieved with a microwave, a pressure cooker, a hot plate, or other type of heating device. Also, in preferred embodiments, the preheat temperature is such that the pretreatment buffer boils. For example, a preferred temperature range is 96-100 degrees Celsius. A particularly preferred temperature range is 98-100 degrees Celsius. It was determined that the temperature range of 98-100 gives enhanced CISH detection results (e.g. as compared to 92 degrees Celsius). The tissue samples are then generally washed (e.g. with water or PBS) two or three times (e.g. for 2-4 minutes per wash).

Generally, the next step is an enzyme digestion step. In preferred embodiments, the tissue samples are exposed to pepsin digestion (e.g. at room temperature or at about 37°C) for about a several minutes (e.g. 1-20 minutes may be required depending on tissue fixation). Importantly, excessive digestion may cause loss of nuclei and chromosome structure, while inadequate digestion may result in loss of signal. The tissue samples are then washed again (e.g. with water or PBS) two or three times (e.g. for 2-4 minutes per wash).

After washing, the tissue samples are then dehydrated with graded alcohols. For example, the tissue samples may be exposed to 70%, 85%, 95%, and 100% ethanol for about 2 minutes each time, and then air dried.

### ii. Denaturation and Hybridization

Denaturation and hybridization may accomplished as one step (co-denaturing and hybridization, described in this paragraph), or as two steps (separate denaturation and hybridization, described below). One general procedure for co-denaturation and hybridization is as follows. First, add the probe (e.g. 12-20 ul of a subtracted probe library) to the center of a cover slip (e.g. 22 x 22 mm coverslip, or 24 x 32 mm coverslip, or coverslips described in W00138848 to Ventana Medical Systems Inc.). In other embodiments, the probe is added directly to the tissue sample. In other embodiments, the liquid COVERSLIP from Ventana Medical Systems, Inc. is applied over the tissue sample (e.g. to create a humid reaction chamber on the slide). In other embodiments, the Zymed CISH UNDERCOVER slips are employed (available from Zymed Labs.). In some embodiments, the coverslip is then placed probe side down on the tissue sample. The edges of the coverslip may then be sealed, for example, with a thin layer of rubber cement to prevent evaporation during incubation. The slide with the tissue sample is then placed on a slide block of PCR machine or on a heating block with temperature display (or other heating device). Denaturation is conducted at approximately 94-95 degrees Celsius for about 5-10 minutes. The tissue sample (e.g. on the slide) is then incubated at approximately 37 degrees Celsius for about 16-24 hours. Incubation may be conducted, for example, in a dark humidity box (or other humidified chamber) or in the slide block of a PCR thermal cycler.

One general procedure for separate denaturation and hybridization is as follows. This procedures is useful, for example, when a PCR machine or heating block are not readily available. First, the tissue sample is denatured in denaturing buffer (e.g. 4 ml 20x SSC [20x SSC buffer = 0.3M Sodium Citrate, with 3M NaCl, ph 7.0], 8 ml ddH₂O, 28 ml formamide) at about 75 degrees Celsius for about 5 minutes. Increases in temperature may be used for additional samples being denatured at the same time (e.g. add about 1 degree Celsius for each additional sample being denatured). Next, the slides are denatured with graded alcohols (e.g. 70% EtOH, 85% EtOH and 95% all for about 2 minutes at negative 20 degrees Celsius, and then 100% EtOH for about 2 minutes twice).

Then the tissue samples are air dried, while the labeled probe (e.g. subtracted probe) is denatured at about 75 degrees Celsius for about 5 minutes. The denatured probe is then placed on ice. About 12-15 ul of the denatured probe is added to the center of a coverslip (e.g. a 22x22 mm coverslip, or other cover). The coverslip is then added to the appropriate tissue sample area, and the tissue sample is placed in a dark humid box (or other humidified chamber) at about 37°C for at least about 14 hours. Next step, for example, would be the stringency wash below.

### B. Cell Sample or Metaphase Chromosome Sample

### i. Pretreatment

Initially, slides may be immersed in a pretreament buffer such as 2x SSC buffer (20x SSC buffer = 0.3M Sodium Citrate, with 3M NaCl, ph 7.0), or Tris-EDTA, or Tris, at about 37 degrees Celsius for about 60 minutes. In some embodiments, the cell samples are treated with pepsin compositions (e.g. Zymed's SPOT LIGHT Cell Pretreatment Reagent) for about 5 minutes at about 37 degrees Celsius. Incubation time may be, for example, from about 1-10 minutes depending on cell type and slide-making conditions. Excessive pepsin digestion may cause loss of nuclei and chromosome structure. Inadequate digestion may result in loss of signal. Slides may then be washed (e.g. in dH₂0 or PBS) for two or three time, for two or three minutes each time at room temperature. In some embodiments, the slides may be immersed in buffered formalin (e.g. 10%) for about a minute at room temperature. The slides may then be washed (e.g. in dH₂0 or PBS) two or three times for about 1-3 minutes each time, at room temperature. The slides may then be dehydrated. For example, the slides may be dehydrated in 70%, 85%, 95%, and 100% ethanol for 2 minutes each, and then air dried. Slides may proceed to ISH procedures described below or stored (e.g. in 70% ethanol at. -20 degrees Celsius).

### ii. Denaturation and Hybridization

First, add the probe (e.g. 12-20 ul of a subtracted probe library, See Subtracted Probe section below) to the center of a cover slip (e.g. 22 x 22 mm coverslip, or 24 x 32 mm coverslip, or coverslips described in W00138848 to Ventana Medical Systems Inc.). In other embodiments, the probe is added directly to the tissue sample. In some embodiments, the liquid COVERSLIP from Ventana Medical Systems, Inc. is applied over the tissue sample (e.g. to create a humid reaction chamber on the slide). In other embodiments, the Zymed CISH UNDERCOVER slips are employed (available from Zymed Labs.). In some embodiments, the coverslip is then placed probe side down on the tissue sample. The edges of the coverslip may then be sealed, for example, with a thin layer of rubber cement to prevent evaporation during incubation. For denaturation, the slide with the tissue sample is then placed on a slide block of PCR machine or on a heating block with temperature display (or other heating device). Denaturation is conducted at approximately 80 degrees Celsius for about 2-5 minutes. The slides may then be placed in a dark humidity box (or other humidity chamber) or in the slide block of a PCR thermal cycler for about 16-24 hours at about 37 degrees Celsius.

### iii. Stringency Wash

The remaining steps (e.g. stringency wash, immunodetection, counterstaining/ coverslipping) are generally the same for both cell sample and FFPE. After hybridization, the rubber cement (or other sealant used, if a sealant is used) and cover slip (or other cover) is carefully removed. The tissue sample slides are then washed (e.g. in Coplinjar) in order to remove unhybridized probes. For example, the tissue sample slides may be washed in 0.5x SSC at 72°C for about 5 minutes. The temperature may be adjusted up if more than one slide is being washed (e.g. add 1°C per slide for more than 2 slides, but preferable no higher than 80°C. The slides are then washed again in, for example, dH₂O or PBS/Tween 20 buffer for about 2-3 minutes. This may be repeated two or three times.

### iv. Immunodetection

Generally, depending on the detection reagents used, the first step in preparation for immunodetection is peroxidase quenching and endogenous biotin blocking. For peroxidase quenching, slides may be submerged in 3% H₂O₂ in absolute methanol (e.g. add part 30% hydrogen peroxide to 9 parts absolute methanol) for about 10 minutes. The slide is then washed with PBS (e.g. 1 x PBS (10mM)/Tween 20 (0.025%)) for 2-3 minutes. This may be repeated two or three times. The tissue samples are then blocked. Blocking can be performed by adding 2 drops per slide (at room temperature) of CAS-BLOCK (which is 0.25% casein, 0.2% gelatin, and 10mM PBS, pH 7.4). After about 10 minutes, the blocking reagent is blotted off.

Next, the labeled probe library is detected. The probe may be detected by first adding an anti-label primary antibody (e.g. a mouse antibody or antibody with a label such as FITC). In certain preferred embodiments, the probe is labeled with digoxigenin, and the primary antibody is an FITC-anti-dig antibody. In other preferred embodiments, the primary antibody is unlabelled, but is from a particular species such as rat, mouse or goat. In other embodiments, the primary antibody is linked (e.g. conjugated) to an enzyme (e.g. horseradish peroxidase (HRP) or alkaline phosphatase (AP)) able to act on a chromogenic substrate, and does not require the secondary antibody described below. Generally, about two drops of the primary antibody solution is added to the tissue at room temperature for about 30-60 minutes. The tissue sample is then rinsed, for example, with PBS (e.g., 1 x PBS/Tween 20 (0.025%) for about 2-3 minutes. This maybe repeated two to three times.

In preferred embodiments, a secondary antibody is added to the tissue sample that is able to bind to the primary antibody. For example, if the primary antibody is labeled with FITC, the secondary antibody may be an anti-FITC antibody. Also for example, if the primary antibody is an unlabeled mouse antibody, the secondary antibody may be an anti-mouse antibody (e.g. goat anti-mouse antibody). Generally, the secondary antibody is linked (e.g. conjugated) to an enzyme (e.g. HRP or AP) able to act upon a chromogenic substrate (or chemiluminescent substrate). Generally, about 2 drops of the secondary antibody is added to the tissue sample at room temperature for about 30-60 minutes. The tissue sample is then rinsed, for example, with PBS (e.g., 1 x PBS/Tween 20 (0.025%) for about 2-3 minutes. This may be repeated two to three times. Additional antibodies (e.g. tertiary, quaternary antibodies) may be used if desired.

In certain preferred embodiments, the secondary antibody is linked to a polymer that is itself linked to many enzyme molecules (e.g. polymerized HRP or polymerized AP). This allows each individual antibody to connect (via the polymer) to many enzyme molecules in order to increase signal intensity. Such polymerized enzymes are known in the art, and are commercially available from, for example, Nichirei Inc. (Tokyo, Japan) and ImmunoVision.

Once the antibody (or other detection molecule) which is linked to an enzyme (e.g. a secondary or tertiary antibody conjugated to AP or HRP), is added to the biological sample, a substrate for the enzyme is then added. In preferred embodiments, the substrate is a chromogen. Examples of suitable chromogens include, but are not limited to, DAB, FAST RED, AEC, BCIP/NBT, BCIP/INT, TMB, APPurple, ULTRABLUE, TMBlue, and VEGA RED. In other embodiments, the substrate is a chemiluminescent molecule (e.g. BOLD APS 540 chemiluminescent substrate, BOLD APS 450 chemiluminescent substrate, or BOLD APB chemiluminescent substrate, all commercially available from INTERGEN Co.). Therefore, the next step, for example in developing the slide, is to mix DAB (or other substrate), buffer, and hydrogen peroxide (e.g. 0.6%) in a tube, then to add 3 drops per slide to the tissue sample for about 30 minutes. In certain embodiments, chromogen enhancers are added to increase signal intensity (e.g. AEC enhancer, FAST RED enhancer, and DAB enhancer available from INNOVEX Biosciences, ZYMED Labs, etc.). The tissue sample may then be washed (e.g. with running tap water) for about two minutes. In certain embodiments, the immunohistochemistry steps are automated or partially automated. For example, the ZYMED ST 5050 Automated Immunostainer may be employed to automate this process.

### v. Counterstaining and Coverslipping

In some embodiments, the next step is a counterstaining and coverslipping step. This step may be performed by counterstaining the tissue sample. For example, the tissue sample may be counterstained with hematoxylin or other counterstain. This procedure may be performed for about 6 seconds to about 1 minutes, depending on the type of tissue being stained. Preferably, overly dark counterstaining is avoided so as not to obscure the positive signal. The slides may then be washed (e.g. with running tap water) for a couple of minutes, and then, in some embodiments, dehydrated with graded EtOH (e.g. 70%, 85%, 95%, 100%, 100% for about 2 minutes each, repeated two times). In some embodiments, the dehydration is not performed with EtOH, when, for example, FAST RED is the substrate (e.g. a water soluble substrate). The slides may then be exposed to Xylene for about two minutes (this may be repeated at least once). The tissue sample may then be coverslippped (e.g. with HISTOMOUNT, Cytoseal 6.0, cat. # 8310-16, Stephen Scientific). In some embodiments, CLEARMOUNT is employed instead (e.g. when FAST RED is one of the substrates).

### vi. Microscopy and Interpretation of Results

Importantly, the slides may be visualized using standard bright-field microscopy using a bright-field microscope (e.g. OLYMPUS, NIKON, LEITZ, etc.). Generally, probes are visible with about 20X magnification (e.g. 15X-25X). In preferred embodiments, probes are visualized with about 30X, or 40X (e.g. 28X-43X) magnification. Higher powers (e.g. 60X, 80X, and 100X) may be employed, but are generally not necessary (and may reduce the field of view). In some embodiments, for evaluating translocation results, a 100X oil lens is employed. In other embodiments, for evaluating amplification and centromere probes, 40X lens is employed. Below are examples of how CISH results may be interpreted for gene amplification/centromere detection, as well as for gene translocation.

As mentioned above CISH detection of gene amplification, translocation, and cetromere detection may be performed with a bright-field microscope, or other type of microscope. For example, in general, CISH staining results are clearly seen using a 40x objective in tissue sections which are counterstained (e.g., hematoxylin). An individual gene or chromosome centromere signal normally appears as a small, single dot Targeted gene amplification is typically seen as large chromogen-stained (e.g. DAB-stained) clusters or many dots in the nucleus or mixed clusters and multiple dots (e.g., ≥6 dots per nucleus). Tumors with no targeted gene amplification typically show 1 to 5 dots per nucleus. Normally, 3-5 dots per nucleus in more than 50% of tumor cells are due to chromosome polysomy. Table 1 shows an exemplary chart useful for CISH visualization of individual genes for chromosome polysomy.

**Table 1**

| **Exemplary CISH Signal Visualization for an individual gene or chromosome centromere** | |
|---|---|
| Magnification | CISH Signal |
| 10x | Individual signals are barely visible and may be missed. |
| 20x | Individual signals are small but clearly discernible. |
| 40x | Individual signals are easily identified. |
| 60x or 100x | Not Necessary |

Examples of CISH detection and interpretation of gene amplification in HER2 and TopoIIα CISH, are presented in Tables 2 and 3 below.

**Table 2**

| **Exemplary Assessment of HER2 gene status by CISH** | |
|---|---|
| Amplification | >10 copies or large clusters of HER2 gene (amplicon) per nucleus in >50% of cancer cells. |
| Low Amplification | 6-10 copies of HER2 gene or small cluster of HER2 gene (amplicon) per nucleus in >50% of cancer cells. |
| | Labeled chromosome 17 centromere probe may be applied for CISH to confirm that 6-10 copies of HER2 gene (<5%.cases) were due to HER2 gene amplification but not chromosome 17 polysomy. |
| No Amplification | 1-5 copies of HER2 gene per nucleus in >50% of cancer cells. |
| | 3-5 copies of HER2 gene per nucleus is due to chromosome 17 polysomy. There is no need for chromosome 17 centromere CISH. |
| | Occasionally, it is found that HER2 has 3-5 copies and chr.17cen has 1-2 copies in >50% of cancer cells (HERZ/chr.17cen ratio is ≥2), it is due to what sometimes was seen by CGH of duplication of chromosome aim 17q. |

**Table 3**

| **Exemplary Topo IIα Probe and Chromosome 17 Centromeric Probe Usage** | | |
|---|---|---|
| **Topo IIα Status** | **Topo IIα Results** | **Chromosome 17 Centromeric Probe** |
| Deletion | When Topo IIα gene copy number is less than the centromeric copy number. | |
| Normal diploid | 2 copies | 2 copies |
| Aneuploidy | 3-5 copies | 3-5 copies |
| Amplification | Gene cluster (amplicon) or ≥6 separate copies | Gene amplification is highly likely, Chromosome 17 Centromeric Probe analysis is not necessary |

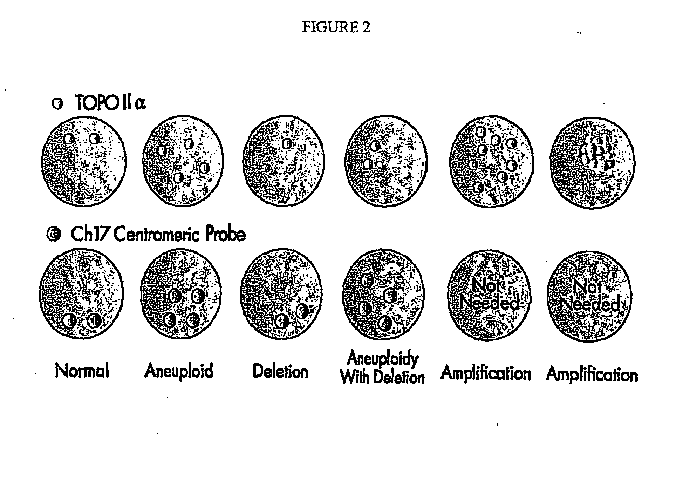

Also, in some normal cells, one gene copy may be missing due to loss of nuclear material during sectioning. Therefore, in general, analysis should be based on the results from the majority of cancer cells (>50%) observed. Figure 2 presents one interpretation chart for interpreting topoIIα amplification using topoIIα and chromosome 17 centromere probes. It should be noted that these are representative examples only. Copy numbers from actual samples may vary for aneuploidy, deletion, and amplification.

### vii. Quality Control Procedures

In some embodiments, quality control procedures are used. Quality control over the accuracy of the above procedures may, in some embodiments, be assured by using some or all of the controls described below.

### Positive Tissue Control:

External positive control materials for clinical research generally should be fresh autopsy/biopsy/surgical specimens fixed, processed, and embedded as soon as possible in the same manner as the patient sample(s). Specimens processed differently from the specimen sample(s) validate reagent performance, and do not verify tissue preparation. Positive tissue controls are indicative of correctly prepared tissues and proper staining techniques. One positive tissue control for each set of test conditions may be included in each run. For example, for topoIIα gene detection, tissues used for the positive control materials should be selected from specimens with well-characterized levels of topoIIα gene. Approximately 5-10% of breast cancer tissue has topoIIα gene amplification and may be a useful source of positive control tissue.

Known positive controls may be utilized for monitoring the correct performance of processed tissues and test reagents, rather than as an aid in interpreting sample results. If the positive tissue controls fail to demonstrate positive staining, results with the specimen samples should generally be considered invalid.

### Negative or Normal (Diploid) Tissue Control:

Normal tissue can be used as a negative control for gene amplification or deletion. Use a negative tissue control (known to be diploid) fixed, processed, and embedded in the same manner as the sample(s) with each staining run. This will verify the specificity of the ISH probe, and provide an indication of non-specific background staining (false positive staining).

A negative tissue control that is separate from the sample is known as an 'external' negative control. If an external negative tissue control is not available then a normal section of the sample can serve as an 'internal' negative tissue control.

In certain embodiments, the negative tissue control is examined after the positive tissue control to verify the specificity of hybridization. Generally, the presence of no more than two gene copies in most of the cells in the negative tissue control confirms that the probe and detection reagents are not cross-reacting with cellular or tissue components. Occasionally, 0,1,3, or 4 gene copies may be seen in the nucleus. Normal tissue counterparts in an abnormal sample may also be used as negative tissue controls. If non-specific straining occurs in the negative tissue control, results obtained for the sample specimen(s) should generally be considered invalid. Also, non-specific staining usually exhibits a diffuse staining pattern. Sporadic staining of connective tissue may also be observed in sections from excessively formalin-fixed tissues. In preferred embodiments, normal cells are used for interpretation of staining results as necrotic or degenerated cells often stain non-specifically.

### Reagent (No-Probe) Control:

A reagent control may be run on a section of sample specimen without the probe. The reagent control is useful in evaluating the possibility of nonspecific staining, particularly when performing ISH in tissue sections. The reagent control may be stained in the same way as the test samples except that hybridization buffer, that does not contain the probe, should generally be used during the hybridization step. Slide pretreatment, denaturation, and immunodetection should generally be performed under the same conditions as test samples.

### viii. Automation

In certain preferred embodiments, all or part of the procedures described above for performing CISH are automated. Automation is useful for high throughput processing of many samples (e.g. in a clinical lab). Examples of methods and devices useful for such automation are found in WO9943434, and WO9944030 to Ventana Medical Systems Inc., (Tucson, AZ). Additional examples are automated in situ hybridization and immunohistochemistry devices commercially available from Ventana Medical Systems Inc, such at the BENCHMARK in-situ hybridization module. In other embodiments, Cytologix Corp. (Cambridge Mass.) equipment is used (e.g. as shown in WO0063670, WO0062064, WO9944032, W09944031, and WO9901770).

### II. CISH HER-2/neu Detection and Anti-HER2 Antibody Therapy

The present invention also provides methods, kits, and compositions for detecting HER2 gene amplification (e.g. on a patient) sample using CISH (See, e.g, Examples 1 and 2). Once HER2 gene amplification is detected by CISH, the patient from which the sample is derived is then able to be identified as a good candidate to receive anti-HER2 antibody immunotherapy. In some embodiments, the patient is prescribed anti-HER2 antibodies. In other embodiments, the patient is administered a therapeutic dose or doses of anti-HER2 antibodies (e.g. chimeric, humanized, or fully human anti-HER2 antibodies).

Examples of antibodies which bind HER2 include, but are not limited to, MAbs 4D5 (ATCC CRL 10463), 2C4 (ATCC HB-12697), 7F3 (ATCC HB-12216), and 7C2 (ATCC HB 12215) (see, US Patent No. 5,772,997; PCT Publication No. WO 98/77797; and US Patent No. 5,840,525). Examples of humanized anti-HER2 antibodies include, but are not limited to, huMAb4D5-1, huMAb4D5-2, huMAb4D5-3, huMAb4D5-4, huMAb4D5-5, huMAb4D5-6, huMAb4D5-7, and huMAb4D5-8 (HERCEPTIN^{®} & commat;) as described in Table 3 ofU. S. Patent 5,821,337; and humanized 520C9 (PCT Publication No. WO 93/21319). Examples of human anti-HER2 antibodies include, but are not limited to, those that are described in U.S. Patent No. 5,772,997 and PCT Publication No. WO 97/00271.

### III. Combined HER2/HER-2/neu and topoIIα detection

The present invention provides methods for diagnosing and treating cancer, and in particular, methods for determining the susceptibility of subjects suspected of having breast cancer (or known to have breast cancer) to treatment with topoisomerase II inhibitors and treatment with anti-HER2 antibody therapy. Importantly, the present invention provides methods, compositions, and kits for detecting copy number for both topoIIα and HER-2/neu, or detecting HER2 expression (e.g. overexpression) and topoIIα copy number (e.g. amplification) which leads to improved diagnostic treatment procedures (e.g. for successfully treating breast cancer patients). For example, given the dangers associated with the co-administration of topoisomerase II inhibitors (such as anthracyclines) and anti-HER2 antibodies (e.g. HERCEPTIN^{®}), the present invention provides methods for selecting which treatment is likely to be useful for a particular patient. This is accomplished, in some embodiments, by determining a copy number for both topoIIα and HER2/neu in a tissue sample from a patient (e.g. breast cancer patient), or detecting a copy number for topoIIα and expression levels of HER2.

In some embodiments, the present invention provides methods for determining whether a subject suspected of having breast cancer would benefit from treatment with topoisomerase II inhibitors (*e.g.*, anthracyclines). For example, the present invention provides diagnostic assays for detecting an amplified copy number of HER-2/*neu* and *topoII*α in breast cancer cells of a candidate subject, and identifying whether the candidate subject is suitable for treatment with topoisomerase II inhibitors (e.g. without concomitant anti-HER2 antibody therapy), or treatment with anti-HER2 antibody therapy (e.g. without concomitant topoisomerase II inhibitor therapy such as anthracycline therapy). In other embodiments, the present invention provides methods for treating breast cancer by administering topoisomerase II inhibitors *(e.g.,* anthracyclines) to subjects, with breast cancer cells with an amplified copy number of HER-2/*neu* and *topoIIα.* For ease in reading, this section is divided into the following sections: A. Breast Cancer, B. Treatment for Metastatic Breast Cancer; C. TopoIIα and *TopoIIα;* D. Detection of *TopoIIα;* E. HER-2 and HER-2/*neu;* F. Detection of HER2 and HER-2/*neu*; G.; HER-2/*neu - TopoIIα* Relationship; and H. HER-2/*neu* - *TopoIIα* Status as Diagnostic Marker.

### A. Breast Cancer

Despite earlier diagnosis of breast cancer, about 1-5% of women with newly diagnosed breast cancer have a distant metastasis at the time of the diagnosis. In addition, approximately 50% of the patients with local disease who are primarily diagnosed eventually relapse with the metastasis. Eighty-five percent of these recurrences take place within the first five years after the primary manifestation of the disease.

On presentation, most patients with metastatic breast cancer have only one or two organ systems involved. As the disease progresses over time, multiple sites usually become involved. Indeed, metastases may be found in nearly every organ of the body at autopsy. The most common sites of metastatic involvement observed are locoregional recurrences in the skin and soft tissues of the chest wall, as well as in axilla, and supraclavicular area. The most common site for distant metastasis is the bone (30 - 40% of distant metastasis), followed by lung and liver. Metastatic breast cancer is generally considered to be an incurable disease. However, the currently available treatment options often prolong the disease-free state and overall survival rate, as well as increase the quality of the life. The median survival from the manifestation of distant metastases is about three years.

In some patients, advanced disease can be controlled with therapy for many years allowing good quality of life. This is particularly evident for those patients with hormone receptor positive disease and nonvisceral sites of metastases. It is contemplated that with better understanding of the molecular factors involved in the response to chemotherapy and increased efficiency of chemotherapy, regimens will substantially extend the survival for these patients, and in some patients, perhaps even extend survival to their otherwise natural life-span. However, despite these promises, the current reality is that treatment provides only temporary control of cancer growth for most patients with metastatic breast cancer.

### B. Treatment for Metastatic Breast Cancer

Systemic drug therapy for advanced breast cancer is usually started with hormonal therapy due to its lower toxicity than the cytotoxic chemotherapies. The best candidates for hormonal therapy, based on their clinical features, are patients with a hormone receptor positive tumor (especially when both hormone receptors are positive), long term disease free survival, previous response to hormonal therapy, and non-visceral disease. Despite short second-line and even third-line responses to alternative hormonal therapies (*e.g*., second anti-estrogen or aromatase inhibitor) in advanced stage of breast cancer, nearly all patients finally become refractory to hormonal therapy and their disease progresses.

Due to its higher toxicity, cytotoxic chemotherapy is given to patients with disease refractory to hormonal therapy. In addition, it is frequently used as the first-line therapy for those with extensive visceral involvement of metastatic disease (*e.g.*, lung or liver metastasis), with hormone receptor negative primary tumor, with extensive involvement of bone marrow, or with tumor that is so rapidly growing that the response to hormonal therapy can not be monitored. Combination chemotherapy for advanced breast cancer is generally considered more efficacious than single-agent therapy. However, randomized trials have shown that similar response rates can be achieved with single-agent therapy.

Advanced breast cancer is currently considered to be incurable and nearly all available chemotherapeutic drugs have been tested for use in its treatment. Among the large number of cytotoxic drugs, anthracyclines (which are topoII-inhibitors), especially doxorubicin and its derivative epirubicin, and taxanes are considered to be the most efficacious. The optimal schedules for the newer drugs, paclitaxel and docetaxel (taxanes), are yet to be established.

In addition to anthracyclines, other topoll-inhibitors include cytotoxic agents such as etoposide, amsacrine, and mitoxantrone. All these agents target the topoisomerase IIα enzyme (topoIIα) and are now routinely employed in the systemic treatment of hematological cancers and solid tumors. Generally, the chemotherapeutic regimens for the most curable malignancies, such as lymphomas and leukemias, as well as for breast cancer are based on such agents that act on topoIIα.

In the treatment of breast cancer, these compounds are not only given for patients with metastatic disease, but are also gaining popularity as a foundation for adjuvant chemotherapy regimens. Whether given alone or combined with other cytotoxic drugs, the objective response rate to anthracyclines generally ranges from 40% to 80% in metastatic breast cancer. However, the rate of complete response is approximately S - 15% and usually lasts for one to two years in these patients. The proportion of patients who achieve complete, prolonged (*i.e*., several years) remissions is below 1%. More typically, the response is partial (50% reduction in tumor mass) and its duration ranges from 6 to 12 months. Thus, there is still a large number of patients who do not receive objective, clinical response to these cytotoxic drugs. In these patients the disease progression may just be halted or continue to progress despite the treatment. About 40 - 60% of the breast cancer patients receiving anthracyclines have either stabilized or develop progressive disease during the therapy. Therefore, there is a need for reliable selection of patients who are likely to respond to therapy from those likely to have primary resistance to anthracyclines.

As important as it is to identify the patients likely to respond to therapy, it may be even more relevant to identify patients who are not likely to achieve any objective response to anthracyclines, because the tumors resistant to anthracyclines also acquire resistance to other classes of cytotoxic drugs during anthracycline therapy (*i.e.,* the tumors become multidrug resistant (MDR)). The MDR phenotype turns cancer cells resistant to virtually any form of cytotoxic chemotherapy (excluding the taxanes). Indeed, MDR tumor cells are even resistant to agents with no functional or mechanistic interaction with topoII-inhibitors.

The most recent breakthrough in the treatment of human malignancies has been the introduction of monoclonal antibodies which specifically target genes that are involved in the pathogenesis of cancer. The first such antibody targeting human oncogene is called Trastuzumab (HERCEPTIN^{®}, Genentech BioOncology, Roche), and was introduced to the treatment of breast cancer patients in 1997. HERCEPTIN^{®} specifically binds the extracellular domain of the HER-2 and abolishes growth factor signaling through HER-2 and other growth factor receptors attached to HER-2.

In clinical trials, HERCEPTIN^{®} was shown to be generally well tolerated with the most common adverse effects being chills and fever in approximately 40% of patients (mainly associated with the first infusion). However, when administered in conjunction with anthracyclines, BERCEPTIN^{®} resulted in an increased risk of cardiac dysfunction in patients. In particular, it has been reported that 27% of patients receiving combined therapy with HERCEPTIN^{®} and anthracyclines experienced cardiac dysfunction, while only 6% of patients receiving anthracycline therapy alone experienced cardiac dysfunction. Thus, the present invention provides methods useful for identifying candidate subjects that would benefit from anthracycline therapy, even though they may initially be viewed as HERCEPTIN^{®} therapy candidates. The present invention also provided methods (e.g. dual topoIIα and HER2/neu testing) useful to identify patients that should receive HERCEPTIN^{®} without also receiving anthracyclines (or other topoIIα inhibitors).

### C. TopoIIα and TopoIIα

Topoisomerases are enzymes involved in resolving topological problems that arise during the various processes of DNA metabolism, including transcription, recombination, replication, and chromosome segregation during cell division. As a result of performing these vital functions, topoisomerases are necessary for the viability of all living organisms.

Topoisomerases are classified into "Type I" and "Type II" based on their catalytic activity. Type I enzymes introduce transient single-stranded breaks into DNA, pass a single intact strand of DNA through the broken strand, and re-ligate the break. Type II enzymes, in contrast, make transient double-stranded breaks in one segment of replicated DNA and pass an intact duplex through the broken double-stranded DNA.

Among different topoisomerase enzymes, type II DNA topoisomerases (topoII) are essential in the segregation of newly replicated chromosome pairs, chromosome condensation, forming chromosome scaffolds, and altering DNA superhelicity. The reaction of transporting the intertwined double-stranded DNA through a double-stranded break favors a "two-gate model". In this model, topoII forms an ATP-operated clamp through which the first segment of DNA binds and which then captures the DNA segment to be transported. Once the transported segment has passed through the break in the bound DNA, it is allowed to leave the enzyme by another gate on the other side of the molecule, while the double-stranded break in the bound DNA is simultaneously re-sealed by the enzyme. Consistent with this biochemical model of the enzyme as an ATP-modulated clamp with two sets of jaws at opposite ends, connected by multiple joint, the crystal structure of topoII reveals a heart-shaped dimeric protein with a large central hole.

The eukaryotic topoII is a homodimeric enzyme that exists in two isoforms in human cells, the major, 170-kd topoIIα and 180-kd topoIIα. These two enzymes share considerable homology (72%) but are products of different genes located in chromosomes 17q21-q22 and 3p, respectively. The functions as well as the expression of these two genes are different. Whereas topoIIα expression is cell cycle-dependent, the β-isoform shows no cell cycle-phase dependency. The most abundant expression of topoIIα takes place at the G2/M-phase of the cell cycle and declines to minimum at the end of mitosis. The exact function of topoIIα is still largely unknown.

TopoIIα has raised considerable clinical interest since it is a molecular target for many antineoplastic and antimicrobial drugs. Among the cytotoxic drugs acting on inhibiting topoII are some of the most important anticancer drugs such as anthracyclines (*e.g.,* doxorubicin, epirubicin, daunorubicin, idarubicin), epipodophyllotoxins (*e.g.,* etoposide, teniposide), actinomycin and mitoxantrone. Although these anticancer drugs share no structural homology, they all act by trapping topoIIα in a covalently bound reversible complex with DNA, termed the 'cleavable complex'. The stabilization of cleavable complexes prevents the religation of the double-stranded breaks. This converts topoIIα into a physiological toxin and introduces high levels of permanent double-stranded breaks that are ultimately detected by cell cycle checkpoint and culminate in cell death by apoptosis.

It has been shown *in vitro* that sensitivity to topoII-inhibitors correlates with the expression level of topoIIα in cancer cells. Cells with low nuclear concentrations of topoIIα protein form fewer topoII-mediated DNA strand breaks and are thus less sensitive to topoII-directed drugs than cells containing high amounts of topoIIα. This relationship was first established by comparing the chemosensitivity of different cell lines to their expression of topoIIα, but more recently the relationship has been confirmed with more specific methods. These studies have shown that sensitive cell lines can be made resistant by transfection of either antisense *topoIIα* mRNA or mutant *topoIIα* cDNA. The transfection of exogenous, wild-type *topoIIα* mRNA, in turn, reverses primary resistance to topoII-inhibitors into sensitivity.

### D. Detection of TopoIIα

Detection of the amplification of the topoisomerase IIa *(topoIIα)* gene may be determine by employing in *situ* hybridization of the invention. Probes for *topoIIα* may be obtained, for example, by screening a P1-library, and confirming the identity of the probe by performing PCR with *topoIIα* specific primers (See, Examples 1 and 3). BAC or PAC clones may also be used for *TopoIIα* probe preparation. In preferred embodiments, a *TopoIIα* probe that is capable of specifically detecting *TopoIIα* gene sequence (without falsely detecting HER2/*neu)* are employed. It should be noted that the *TopoIIα* probes briefly sold by Vysis (Downers Grove, IL), were unable to accurately discriminate between *TopoIIα* and HER2/*neu.* However, the present invention provides such specific probes (*e.g*., the Exemplary probe described in Example 3, and commercially available from Zymed Laboratories).

### E. HER2 and HER-2/neu

The HER-2/*neu* oncogene (also known as *erb*B-2) encodes a 185-kDa transmembrane glycoprotein (HER2), which is a member of the family of epidermal growth factor (EGF) receptor tyrosine kinases (RTK). The HER-2 family of RTKs has four members: HER-1, HER-2, HER-3, and HER-4. The RTKs are cell-surface enzymes consisting of a single transmembrane domain separating an intracellular kinase domain from an extracellular ligand-binding domain. Ligand binding to the extracellular domain induces the formation of receptor dimers (homo- or preferentially hetero-), which are essential for activation of the intrinsic tyrosine kinase activity. This subsequently leads to a recruitment of target proteins, that initiate a complex signaling cascade.

Although a large number of putative candidate ligands (EGF, heparin binding EGF-like growth factor, transforming growth factor-α, amphiregulin, betacellulin, epiregulin and a large family of different neuregulins among others) have been postulated to bind HER-2, none of these peptides binds HER-2 with high affinity. However, EGF-like ligands are bivalent Thus, they are capable of binding their receptors at two different sites; namely high affinity as well as low affinity binding sites. Although HER-2 is not a high affinity receptor for any of the ligands shown to bind ErbBs, it is the preferred low affinity co-receptor for EGF-like ligands. Therefore, it emerges as the preferred dimer-mate for the three other ErbBs, once these primary receptors are occupied by their ligands. Thus, at least 20 growth factors can utilize HER-2 related signaling pathways.

HER-2 is vital in the induction of growth signal by the ligand occupied ErbBs, because in the presence of HER-2: 1) it is the preferred heterodimerization partner for all ligand-binding ErbB RTKs and 2) HER-2-containing heterodimers are also characterized by extremely high growth factor-induced signaling potency and mitogenesis. The high signaling potency of HER-2 containing heterodimers, in turn, is attributed to several specific features: 1) HER-2 reduces the rate of ligand dissociation from its high affinity receptor; 2) HER-2 induces lateral signaling by recruiting and activating other (unoccupied) ErbB receptors; and 3) HER-2 efficiently signals through protein kinases (such as MAP and Jun N-terminal), which are especially potent activators of mitosis. In addition, HER-2-containing receptor dimers are recycled from endosomes back to the cell surface instead of being degraded by lysosomes. Thus, these dimers may be overrepresented at the cell surface.

Due to these features, the HER-2 receptor has an oncogenic potential that may be activated through multiple genetic mechanisms including point mutations, truncation of the protein, and the amplification of the non-mutated proto-oncogene. However, gene amplification is by far the most common mechanism for the activation of the oncogenic potential of HER-2. The amplification of HER-2/*neu* happens in approximately 20 to 35 % of invasive breast cancers and results in overexpression of the protein. Thus, the amplification of HER-2/*neu* increases the likelihood of HER-2 to form heterodimeric complexes with the other ErbBs. This, in turn, indicates that several dozen potentful ligands can take advantage of HER-2 dependent signaling pathways leading to the oncogenic activation of cells.

The association of HER-2/*neu* and the prognosis for breast cancer patients has been extensively studied (*e.g*., Ravdin and Chamness, *Gene,* 159:19-27, [1995]). Unfortunately, amplification of HER-2/*neu* has been found to be associated with poor clinical outcome. However, whether HER-2/*neu* is an independent prognostic factor is still controversial because both supportive and non-supportive results have been published (*e.g.*, Ravdin and Chamness, *supra*).

The most common activation mechanism for HER-2/*neu* is by the amplification of the gene at 17q12-q21. The extra copies of HER-2/*neu* oncogene are deposited in cancer cells as extrachromosomal double minute chromosomes or within the chromosomes in homogeneously staining regions.

The predictive value of HER-2/*neu* has also been studied, although not as extensively as its prognostic value, in conjunction with both adjuvant chemotherapy and in chemotherapy for advanced breast cancer (*e.g*., McNeil, *C., J. Natl. Cancer Inst.,* 91:100, [1999]). HER-2/*neu* appears to be a predictor for poor clinical outcome in adjuvant chemotherapy by conventional cyclophosphamide-methotrexate- fluorouracil -combination. The relationship of amplified HER-2/*neu* and topoII-inhibitor chemotherapy in breast cancer is more controversial. Most studies have linked amplified HER-2/*neu* to chemoresistance to topoII-inhibitors (*See, e.g.,* Tetu *et al., Mod. Pathol.,* 11:823 [1998]), but there are also clinical trials reporting either no association (*See, e.g.,* Clahsen *et al., J. Clin. Oncol.,* 16:470 [1998]), or even tendency for higher response rates among HER-2/*neu*-amplified breast tumors (*See, e.g.,* Thor *et al., J. Natl. Cancer Inst.,* 90:1346 [1998]). The results presented in Example 5 of WO 03/025127 support the conclusion that HER-2/*neu* amplification is not associated with clinical response to topoisomerase II inhibitors.

### F. Detection of HER-2 and HER-2/neu

As noted above, HER-2/*neu* oncogene amplification and its concomitant protein overexpression are currently implicated as an important prognostic biomarker in breast carcinoma, and may also be a useful determinant of response to hormonal or cytotoxic chemotherapy. The clinical importance of HER-2/*neu* diagnostics has become even more significant with the increasing use of the new anti-cancer drug trastuzumab (HERCEPTIN, a humanized monoclonal antibody against the extracellular part of the HER-2/*neu* protein product). However, trastuzumab therapy is effective only in patients whose tumors contain amplification and/or overexpression of HER 2 (Shak. S., *Semin. Oncol.,* 6:71 [1999]). Thus, HER-2 assays are now becoming an important part of breast cancer diagnostics, in parallel with assays of hormone receptors and tumor proliferation rate.

The earliest studies of HER-2 used Southern and Western blotting for detection of HER-2/*neu* gene amplification and HER-2 protein overexpression. However, these methods are not well-suited for routine diagnostics and have been replaced by immunohistochemistry and fluorescence *in situ* hybridization (FISH). In addition, a vast majority of HER-2 studies have been done using immunohistochemistry (IHC), which detects the HER-2 protein overexpression on the cell membrane. Without HER-2/*neu* oncogene amplification, the protein expression is generally low and undetectable by IHC. However, IHC is subject to a number of technical artifacts and sensitivity differences between different antibodies and tissue pretreatments. Standardized reagent kits have recently been introduced (*e.g*., HERCEP-TEST, DAKO Corp.), but mixed results have been reported from their methodological comparisons (Jiminez *et al., Mod. Pathol.,* 13:37 [2000]). Other HER-2 commercially available antibodies include two monoclonal antibodies from Novocastra Laboratories, clone CB-11 and NCLB12, and the antibodies described above in section II.

Fluorescent *in situ* hybridization (FISH) quantifies the number of gene copies in the cancer cell nucleus. Since the initial experiments to detect HER-2/*neu* amplification by FISH, a number of reports have verified its accuracy both in freshly frozen and paraffin-embedded tumor material (Mitchell, M.S., *Semin. Oncol.,* 26:108 [1999]). FISH is generally performed using either single-color (HER-2/*neu* probe only) or dual-color hybridization (using HER-2/*neu* and control probes (*e.g*., chromosome 17 centromere probes simultaneously), with the latter method making it easier to distinguish true HER-2/*neu* amplification from chromosomal aneuploidy. FISH using entire cells (*e.g.,* cultured cells, pulverized tissue, or imprint touch specimens from tumors) is considered straightforward, but the use of tissue sections complicates the quantitative nature of FISH due to nuclear truncation (*i.e.,* due to the slicing of the tissues during their preparation for staining). Commercially available FISH probes include Zymed's SPOT-LIGHT HER-2/*neu* probe (Zymed Laboratories, San Francisco, CA), and Vysis's LSI HER-2/*neu* SpectrumOrange probe (Vysis, Downer's Grove, IL).

The main difficulty in adopting FISH for clinical diagnostic use is the requirement for fluorescence microscopy. Evaluation of FISH samples generally requires a modem epifluorescence microscope equipped with high-quality 60x and 100x oil immersion objectives and multi-bandpass fluorescence filters. Moreover, because the fluorescence signals fade within a few weeks, the hybridization results usually must be recorded with expensive CCD cameras.

One aspect of the present invention circumvents many of these problems by providing methods and compositions for detecting HER-2/*neu* that are rapid and do not require the use of fluorescence microscopy. In particular, the present invention provides Chromogenic *In Situ* Hybridization (CISH) HER-2/*neu* detection probes and methods (*See*, Examples 1, 2 and 3) that allow enzymatic detection of HER-2/*neu*. As described in these examples, the present invention provides HER-2/*neu* probe libraries capable of detection by bright field microscopy. Such probes and detection reagents are commercially available from Zymed Inc. (South San Francisco, CA). Another advantage of the HER-2/*neu* probe is the ability to perform CISH and histopathology simultaneously on the same tissue sample (*See,* Example 3). A further advantage of using CISH is the ability to view CISH signal and cell morphology at the same time.

### G. HER-2/neu - TopoIIα Relationship

The relationship between HER-2/*neu* and *topoII*α amplification has been previously studied. Indeed, *topoIIα* has been found to be amplified in breast tumors with HER-2/*neu* amplification [*e.g*., Smith *et al., Oncogene,* 8:933 (1993)]. As *TopoIIα* and HER-2/*neu* are located so close to each other on chromosome 17, that a simple molecular mechanism for this phenomenon previously hypothesized involves amplification of the chromosomal segment bearing both genes [Murphy *et al., Int. J. Cancer,* 64:18-26 (1996), Hoare *et al., Br. J. Cancer,* 75:275 (1997)]. This should lead to similar gene copy numbers for HER-2/*neu* and *topoIIα.* However, during work by the present applicant imbalanced copy numbers for HER-2/*neu* and *topoIIα* were found by employing fiber FISH analysis. The presence of two separate amplicons for closely situated genes such as HER-2/*neu* and *topoIIα* was unexpected.

The relationship between HER-2l*neu* and *topoIIα* amplification and the response of breast cancer cell lines to topoisomerase inhibitors has also been previously been studied. For example, one group reported that a breast cancer cell line with amplification of both HER-2/*neu* and *topoIIα* was the most sensitive to *m*-AMSA and mitoxantrone. [Smith *et al., supra*]. Subsequent to the breast cancer cell line work, the effect of topoisomerase inhibitors on primary breast cancer cells was evaluated in primary breast cancer cells determined to have amplified HER-2/*neu* and *topoIIα* [Jarvinen, *et al., British Journal of Cancer,* 77(12):2267 (1998)]. However, instead of confirming the results previously reported for breast cancer cell lines, the primary breast cancer cells with amplification of both HER-2/*neu* and *topoIIα* were not found to exhibit a positive response to topoisomerase inhibitors.

### H. HER-2/neu - TopoIIα Status as Diagnostic Marker

There are disclosed herein methods for determining whether a candidate subject is suitable for topoisomerase II inhibitor treatment or anti-HER2 immunotherapy by detecting copy number amplification of both HER-2/*neu* and *topoIIα.* There are disclosed methods for identifying a candidate for topoisomerase II inhibitor treatment by providing a candidate subj ect suspected of having breast cancer cells and detecting a copy number for both HER-2/*neu* and *topoIIα* in the breast cancer cells. In this regard, the method allows identification of the candidate subject as suitable for treatment with a topoisomerase II inhibitor by demonstrating amplification of the copy number for both the *HER-2*/*neu* and the *topoIIα.* In some embodiments, the candidate subject has breast cancer cells comprising an amplified copy number for HER-*2*/*neu. (e.g.,* HER-2/*neu* amplification was already determined). In other embodiments, the candidate subject is determined to have HFR2 gene amplification, but not topoIIα gene amplification. These subjects, in some embodiments, are administered anti-HER2 immunotherapy (e.g. BERCEPTIN) without concomitant topoisomerase II inhibitors (e.g. such that the elevated risk of cardiovascular side effects from combined HER2 immunotherapy and anthracycline administration is avoided). In other words, subjects found to have an amplified HER2 gene copy number, but not an amplified topoIIα gene copy number, are identified as suitable for topoisomerase II inhibitor-free (e.g. anthracycline-free) anti-HER2 antibody therapy (i.e. the subject is not administered both topoisomerase II inhibitors and anti-HER2 antibodies around the same time in order to avoid, for example, cardiac problems found in patients given the combination therapy).

In certain embodiments, the detecting is performed with HER-2/*neu* and *topoIIα* specific probes. It is believed that detecting the nucleic acid of *topoIIα* instead of the expressed protein product (*e.g.,* by immunohistochemistry) allows amplification of both HER-2/*neu* and *topoIIα* to serve as a diagnostic marker for breast cancer cells susceptible to treatment with topoisomerase II inhibitors. In particular, there is a lack of correlation between *topoIIα* gene status and immunohistochemical (IHC) detection. Consequently, assessment of *topoIIα* gene expression using IHC detection fails to yield a relationship between amplification of both *topoIIα* and HER-2/*neu* in regard to predicting the response of primary breast cancer cells to topoisomerase II inhibitors. Thus, there is disclosed a breast cancer marker for response to anthracycline based therapy by detecting copy number for both HER-2/*neu* (*e.g*., employing IHC or nucleic acid probes) and *topoIIα* (*e.g.,* employing nucleic acid probes). As such, there are disclosed improved methods for identifying breast cancer patients suitable for treatment with topoisomerase II inhibitors, as well as patients that should not receive topoisomerase II inhibitors (*e.g*., anthracycline). In this regard, patients that are candidates for anti-HER2 immunotherapy (e.g. have increased HER2 expression and/or increased HER2 gene amplification), but do not have amplification of the topoIIα gene (e.g. unlikely to benefit from anthracycline administration) may be administered anti-HER2 antibodies without risking the side effects of anthracylines, and the increased risk of cardiovascular problems, by not administering anthracyclines to these patients.

Importantly, the disclosed methodology allows assessment of patients found to have HER 2/neu amplification *(i.e.,* an indicator for HERCEPTIN^{®} treatment). Indeed, testing to determine whether anthracycline treatment is appropriate (amplification of both HER-2/*neu* and *topoIIα*) or if HERCEPTIN^{®} treatment is appropriate (only HER-2/*neu* amplification). This capability is of particular importance in view of the human trials that have identified serious risks associated with co-administering both anthracyclines and HERCEPTIN^{®}.

### IV. Combined CISH and IHC

There are further disclosed herein methods, compositions, and kits for combined chromogenic in-situ hybridization (CISH) and immunohistochemistry (IHC). The combined CISH and IHC methods allow for comprehensive and valuable information regarding a tissue sample (and patient status) to be determined. In certain embodiments, CISH and IHC are performed on the same tissue sample (e.g. on same part of tissue sample or adjacent sections). In other embodiments, CISH and IHC are performed on different tissue samples, but the tissue samples are from the same biological sample (e.g. from the same breast cancer biopsy sample). In particular embodiments, CISH and IHC are performed simultaneously or nearly simultaneously (e.g. on the same tissue sample or separate tissue samples from the same biological sample). In preferred embodiments, CISH is performed first and IHC is performed second.

In other preferred embodiments, a biological sample is tested by both CISH and IHC (e.g. in order to confirm the presence or absence of HER2 gene amplification and HER2 over expression). In certain embodiments, a tissue sample (e.g. breast cancer biopsy sample) is tested for HER2 gene amplification by CISH and HER2 overexpression by IHC prior to administering (or recommending) anti-HER2 antibody therapy (e.g. HERCEPTIN therapy).

In certain embodiments, CISH and IHC are performed on the same tissue section. For example, the first few steps of CISH may first be performed on the tissue section (e.g. pretreatment, hybridization, and wash). Then, the second party of CISH and IHC may be performed at, or about, the same time. For example, different antibodies may be used. For example, the CISH antibodies may be raised in a first species (e.g. mouse), and the IHC antibodies by be raised in a second species (e.g. rabbit). Also, the detection enzymes used for CISH and IHC may be different, such that the signals can be evaluated individually. For example, the CISH antibodies may be conjugated to HRP, while the IHC antibodies may be conjugated to AP. In this regard, the CISH methods may use a substrate such as DAB, while the IHC methods may use a different substrate such as FAST RED.

### V. Subtracted Probes

The present invention involves subtracted probes (e.g. subtracted probe libraries) useful for CISH. In certain embodiments, the probe libraries are substantially free of repeating sequences (e.g. ALU and LINE elements). For example, in some embodiments, the probe libraries have at least 90% of the repeat sequences removed (e.g. the probe libraries comprise 10% or less of repeat sequences). In other embodiments, the probe libraries have at least 95% of the repeat sequences removed (e.g. the probe libraries comprise 5% or less of repeat sequences). The CISH methods of the present invention are practiced with subtracted probe libraries. Importantly, in certain embodiments, the use of subtracted probes allows a clear signal to be obtained when performing CISH methods (e.g. on cancer biopsy samples).

In some embodiments, the subtracted probe libraries are prepared substantially as described in W00026415 to Fletcher et al.. In certain embodiments, the subtracted probe libraries are performed according to the following procedure. First, clones (e.g. YACs, BACs, or PACs) are chosen that span a gene of interest, or that are on either side of a gene of interest in the case of probe pair libraries. Next, the clone is broken down (e.g. by sonication) into smaller pieces (e.g. 0.1 - 8kb fragments) to form the probe library. Then, in certain embodiments, adapters are ligated on the ends of the fragments (or in some embodiments, adapters are not employed). Next, PCR is performed on the fragments (e.g. using primers specific for the adapters, or random primers). Next, gel size and purification is performed to select a library of fragments in a given range (e.g. 0.5 - 4kb). After that, a subtraction step is performed with labeled repeat (driver) nucleic acid (e.g. biotin labeled COT-1 DNA). The labeled driver nucleic acid is then allowed to hybridize with the library of fragments (tracer nucleic acid). The driver nucleic acid will hybridize to fragments containing complementary repeat sequences (and generally not hybridize to fragments that do not contain these repeat fragments). The mixture is then exposed to a solid support (e.g. beads) conjugated to a second label specific for the label on the driver nucleic acid. In this regard, the driver nucleic acid and the fragments containing repeat sequences hybridized to the driver nucleic acid, are removed from the reaction solution. As a result, the remaining library of fragments has most of the repeat sequences physically subtracted out. The remaining subtracted library may be subjected to further rounds of PCR (e.g. 3 additional rounds of PCR), and then labeled with a desired label (e.g. digoxigenin). The subtracted probe library may then be used in in-situ hybridization procedures, and generally, does not require a blocking step (e.g. the probes dont have to be blocked with repeat sequences, and the tissue sample also does not have to be blocked with repeat sequences).

In some embodiments, the present invention uses a HER2 gene probe library (See, e.g., Example 1). In preferred embodiments, this probe library comprises 90%, preferably 95% repeat free fragments. In some embodiments, the HER2 gene library is specific for the HER2 gene, and is capable of detecting HER2 gene amplification. In particular embodiments, the present invention provides a topoIIα gene probe library (See, e.g., Example 3). In preferred embodiments, this probe library comprises 90%, preferably 95% repeat free fragments. In other embodiments, the topoIIα gene library is specific for the topoIIα gene (e.g. does not falsely detect HER2 gene amplification), and is capable of detection TopoIIα gene amplification. In additional embodiments, the present invention uses an EGFR probe library (See, e.g., Example 5). In preferred embodiments, this probe library comprises 90%, preferably 95% repeat free fragments. In some embodiments, the EGFR probe library is specific for the EGFR gene, and is capable of detecting EGFR gene amplification (by CISH). In other embodiments, the present invention uses an N-MYC probe library (See, e.g., Example 7). In certain embodiments, the N-MYC probe library comprises 90%, preferably 95% repeat free fragments. In some embodiments, the N-MYC probe library is specific for the N-MYC gene, and is capable of detecting N-MYC gene amplification.

In some embodiments, the present invention uses subtracted library probe pairs for detecting gene translocations. In certain embodiments, the probe pairs are "split-apart" probe pairs and are configured hybridize to the centromeric and telomeric regions out side of the breakpoints of targeted genes (e.g. ABL and SYT genes). Additional details on ABL split apart probe pairs, and disease detection are provided below in section VL The breakpoints are located between the gap of the centromeric and telomeric probes such that all (or most) translocations are detected. Split-apart probe pairs, in a normal cell without translocation, show two pairs of dots (e.g. dot has two dots in juxtaposition). The two dots in each pair, for example, shows two different colors in CISH. Also with split-apart probes, a cell with translocation also shows 2 pairs of dots. One pair has two dots in juxtaposition representing the normal chromosome in the cell, the other pair dots are separated representing the translocated chromosome in the cell.

### VI. ABL Probe Pairs and Detecting BCR-ABL Translocations

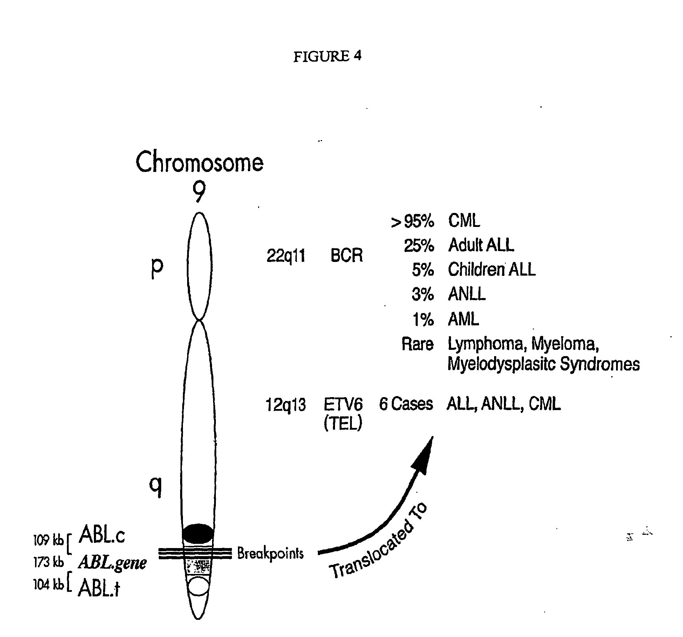

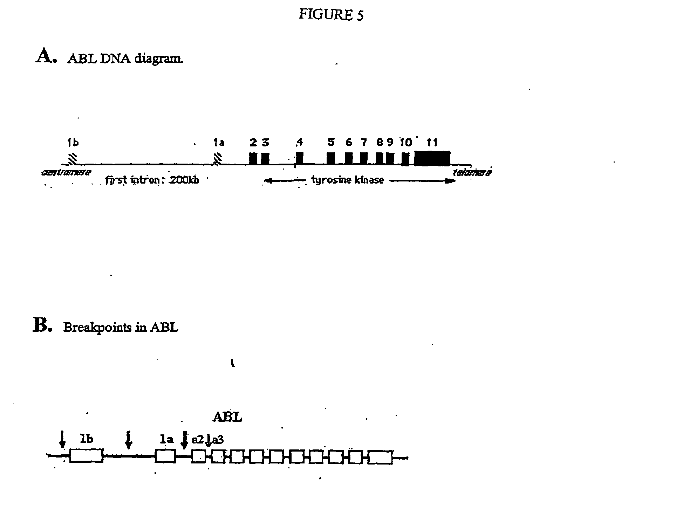

As mentioned above, ABL probe pairs may be used in the invention, for example, to detected BCR-ABL translocations. One example of how to prepare the split-apart ABL probe pair is provided in Example 6. The labeled ABL probe pair can detect BCR-ABL translocation by ISH (e.g. CISH or FISH) on cells from, for example, chronic myeloid leukemia (CML). The staining pattern in these tumor cells is distinctively different from that in normal cells. Translocations involved in the ABL gene (figure 4) are found, for example, in CML, acute lymphoblastic leukemia (ALL), acute non-lymphocytic leukemia (ANLL), and acute myeloid leukemia (AML). Breakpoints in the ABL gene is variable over a region of about 200 kb (figure 5). The ABL translocation probe pairs are, in some embodiments, able to detect all types of the ABL translocations reported so far. Examples of ABL translocations the ABL probe pairs are able to detect are described below.

### i. ABL (Abelson marine leukemia oncogene) gene and protein

The ABL protooncogene spans about 230 kb of genomic DNA, has 12 exons (figure 5), and expressed as either 6 or 7 kb mRNA transcript, with alternatively spliced first exons, exon 1b and 1a, respectively, spliced to the common exons 2-11. Exon 1 b is approximately 200 kb 5-prime of exon 1a (figure 5). The very long intron is a target for translocation in leukemia (see, Bernards et al., 1987, Molec. Cell. Biol. 7:3231-6, herein incorporated by reference). Breakpoints in the ABL gene are variable over a region of about 200 kb (figure 5), often occurring between the two alternative exons 1b and 1a, sometimes 5' of 1b or 3' of 1a. The breakpoint in the intron between exons a2 and a3 of the ABL gene is rarely found.

The ABL gene maps to chromosome band 9q34.1. ABL as well as BCR gene regions have extremely high density, 39.4% and 38.83% respectively, of Alu homologous regions (See, Chissoe et. al, 1995, Genomics, 27:67-82). The 145 kD ABL protein is homologous to the tyrosine kinase (SH1) and regions 2 and 3 (SH2, SH3) of SRC (the chicken Rous sarcoma virus). ABL protein, like SRC, is a non-receptor tyrosine kinase and it has weak enzymatic activity. ABL protein is ubiquitously expressed and expression is located mainly in the nucleus to bind DNA but can migrate into the cytoplasm. Both ABL and transforming ABL proteins inhibit cell entry into S phase by a mechanism that requires nuclear localization and is p53 and pRb dependent (See, Welch and Wang, 1993, Cell, 75:779-790). Interaction of ABL protein with pRb can promote E2F1-driven transcription, for example, of Myc. Alterations of ABL by chromosomal rearrangement or viral transduction lead to malignant transformation, as in CML. Activity of ABL protein is negatively regulated by its SH3 domain, and deletion of the SH3 domain turns ABL into an oncogene.

### ii. BCR (Breakpoint Cluster Region) gene and protein

The BCR gene spans about 130 kb of genomic DNA, has 23 exons and maps to chromosome band 22q11.2. It is proximal to EWS and NF2 genes, both in 22q12. Three breakpoint cluster regions have been characterized to date: major (M-bcr), minor (m-bcr) and micro (m-bcr). Breakpoint in M-bcr, a cluster of 5.8 kb, is between exons 12 and 16, also called b1 to b5 of M-bcr. Breakpoint in m-bcr is in a 35 kb region between exons 1 and 2. Breakpoint in m-bcr is in intron 19. The 160 kD BCR protein has serine/threonine protein kinase activity. BCR protein is widely expressed in many types of human haematopoietic and non-haematopoietic cells and cell lines.

### iii. CML

CML is a malignant clonal disorder of pluripotent hematopoietic stem cells resulting in an increase of myeloid, erythoid, and platelet cells in peripheral blood, and myeloid hyperplasia in the bone marrow. CML is an insidious cancer. It starts out as a genetic flaw spurring overproduction of platelets and white blood cells. Early symptoms are surprisingly few and mild, such as chills and malaise. The typical symptoms of the disease are splenomegaly, fatigue, anorexia and weight loss. Over a few years, the genetically defective cells slowly accumulate more mutations. Eventually the proliferating malignant cells crowd out good cells and the body can no longer fight infection. Though the median age of CML patients is 53 years, the disease also occurs in children. The annual incidence of CML is 10/106 (from 1/106 in childhood to 30/106 after 60 yrs). The disease progresses from the benign chronic phase, usually through an accelerated phase, to the fatal blast crisis within 3-4 years. In contrast to the chronic phase, leukocytes in the blast crisis fail to mature and they resemble myeoblasts or lymphoblasts in patients with acute leukemias. This progression is likely related to the genetic instability induced by BCR-ABL, and is commonly associated with the acquisition of additional, and frequently characteristic, genetic changes. The Ph chromosome and BCR-ABL fusion, however, persist through all phases.

Approximately 4500 new cases of CML occur in the United States each year. The only cure for CML which afflicts 25,000 adults in the U.S. is a bone marrow transplant. But 80% of patients can't find a suitable donor or are too old to risk a transplant. The procedure costs about $150,000 and kills up to 25% of those who undergo it. Drug therapy with alpha-interferon only slows the disease and side effects are so severe that many can't bear it Fortunately, a newly developed drug has been developed by Novartis called GLEEVECA (STI571). GLEEVECA is a small molecule inhibitor of ABL, the first leukemia drug designed to attack the molecular machinery that drives the disease. In one experiment using STI571, 56% of 290 patients who had given up on other therapies enjoyed a partial or complete elimination of cancer cells from their bone marrow. For some patients, traces of cancer can no longer be detected even with exquisitely sensitive DNA probes. Although STI571 has produced complete responses in CML, resistance in some patients highlights the need for other drugs. According to research by investigators at Memorial Sloan-Kettering Cancer Center and Rockefeller University, a new drug called PD17 demonstrated significantly greater potency than STI571 against BCR/ABL containing cell lines and CML patient's cells. PD17 is a member of a class of tyrosine kinase inhibitors originally synthesized by Parke Davis and shown to be potent inhibitors of src family kinases. In addition, another experiment showed that combination of STI571 with adaphostin induced more cytotoxicity in vitro than either agent alone (See, Mow BMF, et al., Blood, 99:664-71, 2001, herein incorporated by reference).

### iv. BCR-ABL translocation

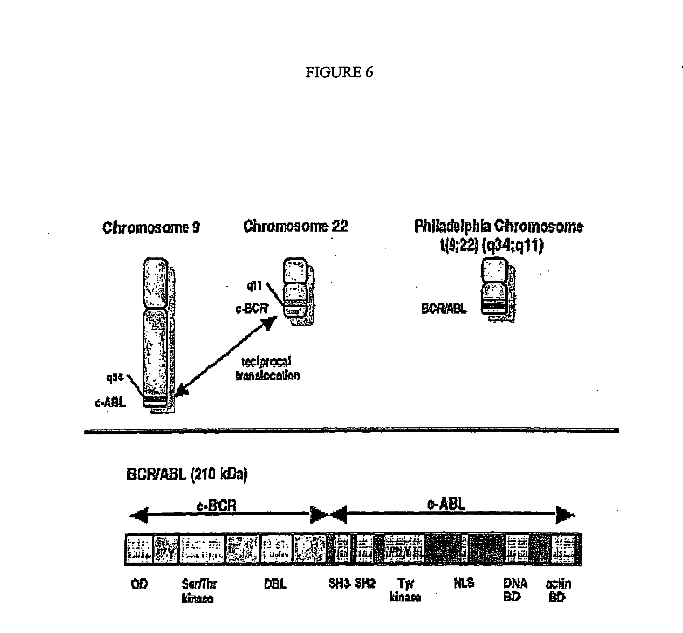

CML was the first malignancy shown to have an acquired and specific genetic abnormality, with the identification of the Philadelphia (Ph) chromosome in 1960, an abnormally shortened chromosome 22. It was demonstrated later (1973) that the Ph chromosome resulted from a reciprocal t(9;22) translocation (Figure 6). The molecular correlates of this translocation were first identified in 1983, with subsequent recognition of the fusion of two distinct genes, BCR and ABL (See, De Klein et al., Nature, 330:765-767, 1982, herein incorporated by reference), resulting in the head-to-tail fusion of the BCR and ABL genes (See, Chissoe et. al, Genomics, 27:67-82, 1995, herein incorporated by reference) (Figure 6).

The BCR/ABL fusion protein greatly increases ABL's tyrosine kinase activity. Its ability to cause CML was demonstrated by retrovirally-transfecting BCR-ABL into mice in 1990, which led to the induction of a CML-like syndrome (See, Scott et al., PNAS, 88:6506-6510, 1991, herein incorporated by reference). While the precise subcellular pathways through which these ultimate biological consequences are attained remain to be definitively dissected, the fact that BCR-ABL is indeed the cause of CML appears clear now, based on the clinical response to targeted tyrosine kinase (TK) inhibition with the drug imatinib mesylate (formerly known as STI571), trade-named GLEEVACA.

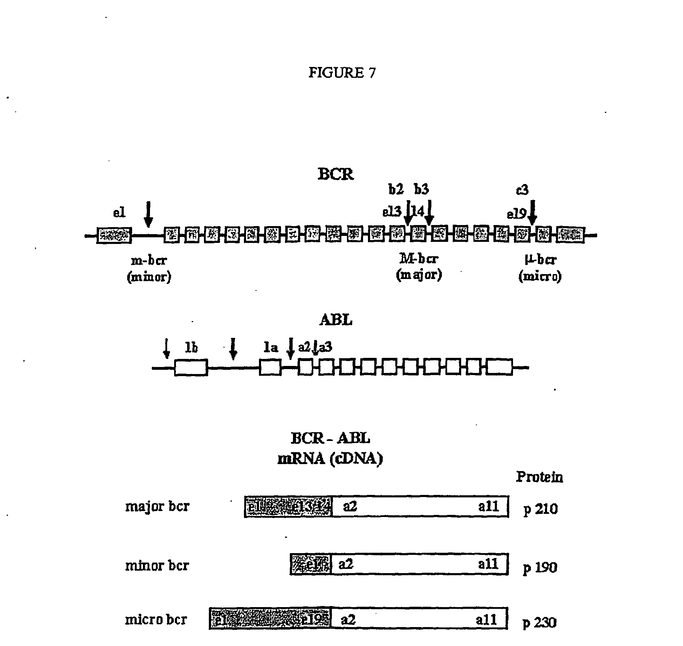

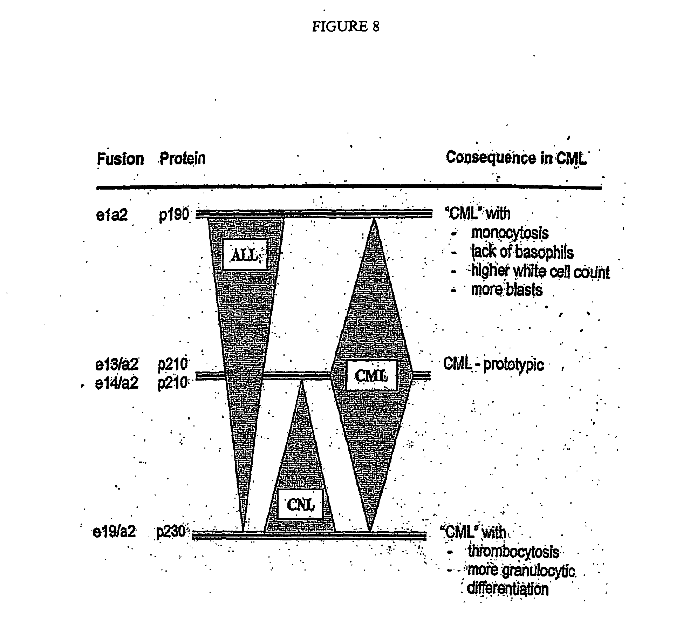

The BCR-ABL hybrid gene, the main product of the t(9;22)(q34;q11) translocation, is found in >95% of CML patients. The BCR-ABL translocation is not exclusive to CML and it is also present in a minority of some other oncohematological diseases, e.g. in about 25% of adult and 5% of children ALL, 1% AML, and very rarely in lymphoma, myeloma or myelodysplastic syndromes. The presence of the BCR/ABL gene does not generally have a diagnostic significance in these diseases but, at least in ALL, it has a prognostic importance, i.e. it is a negative prognostic factor. The fusion protein encoded by BCR-ABL varies in size, depending on the breakpoint in the BCR gene. Three breakpoint cluster regions (figure 7) have been characterized to date: major (M-bcr), minor (m-bcr) and micro (m-bcr) (Melo JV, Baillieres, Clin. Haematol. 10:203-222, 1997, herein incorporated by reference). The overwhelming majority of CML patients have a p210 BCR-ABL gene (M-bcr), whose mRNA transcripts have a b3a2 and/or a b2a2 junction (figure 8). The smallest of the fusion protein, p190BCR-ABL (m-bcr breakpoint), is principally associated with Ph-positive-ALL (Fainstein et al., Nature, 330:386-388,1987, herein incorporated by reference). CML resulting from p230 BCR-ABL gene (m-bcr breakpoint) is also rare. The micro breakpoint has been associated mainly with a mild form of CML, defined as Philadelphia chromosome-positive neutrophilic-chronic myeloid leukemia (Ph-positive CML-N). Exceptional CML cases have been described with BCR breakpoints outside the three defined cluster regions, or with unusual breakpoints in ABL resulting in BCR-ABL transcript with b2a3 or b3a3 junctions, or with aberrant fusion transcripts containing variable lengths of intronic sequence inserts (Melo, supra).

Approximately 5-10% of patients with CML have deletion of the 5' region of ABL and the 3' region of the BCR gene on 9q+ chromosome. The deletions at 5' region of ABL gene, in many cases, can span several megabases. The evidence suggests that these large deletions are associated with a poor prognosis of CML. ETV6-ABL translocation, t(9;12)(q34;q13) are reported in 6 cases of ALL, ANLL and CML (Andreasson P, et aL, Genes Chromosome Cancer, 20:299-304, 1997; Hannemann JR, et al., Genes Chromosomes Cancer, 21:256-259, 1998, both of which are herein incorporated by reference). The breakpoints involved in EWS translocation in chromosome 22 is distal to that in translocation (8;22)-positive Burkitt lymphoma and that in translocation (9;22)-positive chronic myeloid leukemia.

### iv. Split Apart ABL probes

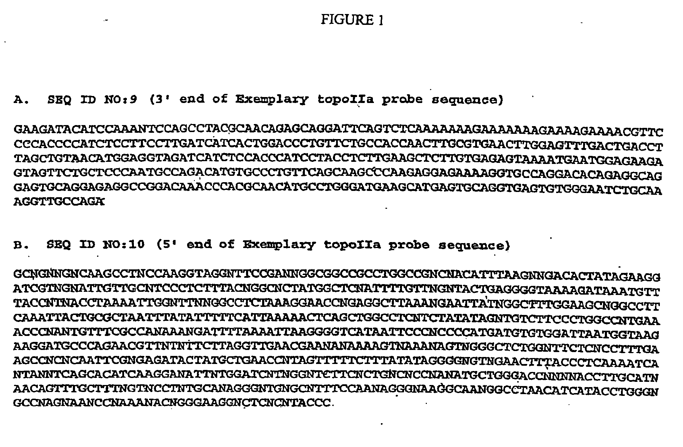

In some embodiments, the present invention uses split apart ABL probe pairs that are able to hybridize to both the centromeric and telomeric regions outside of the ABL gene. In preferred embodiments, the ABL probe pair comprises a probe set configured to hybridize to a region that is centromeric of the ABL gene, and a probe set that is configured to hybridize to a region that is telomeric of the ABL gene. The probe sets comprise subtracted nucleic acid fragments (e.g. less than 90 or 95% or repeat sequences are present), and are detectably labeled. The ABL probe pairs may be employed to detect ABL translocations (see above and figure 4) in order to diagnose a patient suspected of having this type of disease. Example 6 provides one example of ABL split apart probe pairs may be generated. In preferred embodiments, the ABL probe pair is configured such that all the breakpoints in the ABL gene are located between the gap of centromeric and telomeric probes. The ABL probe pair may be used in the CISH method of the invention in order to screen patient samples for ABL rearrangements (e.g. See, Figure 1). The ABL probes should also allow localization of previously uncharacterized translocation partners.

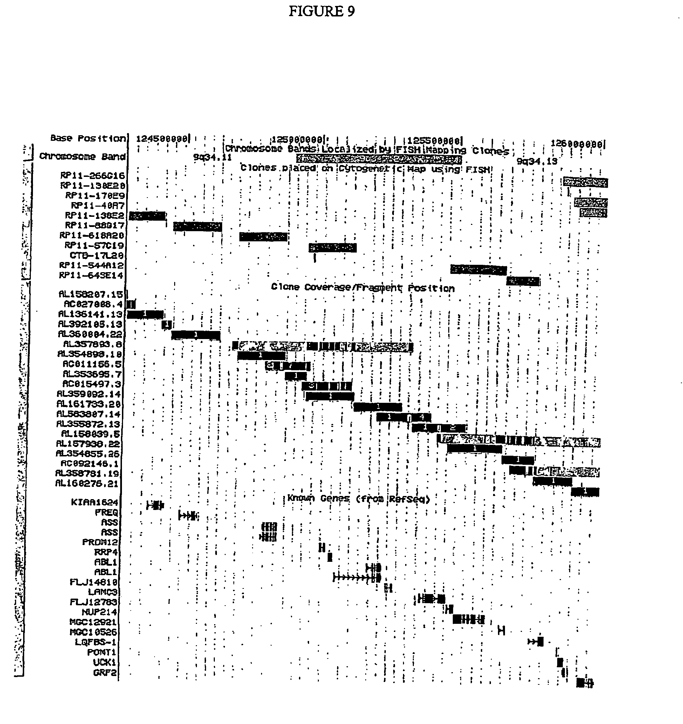

The ABL probe pairs may be generated, for example, as described in Example 6. Also, additional starting clones (e.g. BACs, YACs) selected using computer databases (e.g. human genome sequence information available on the internet) to select sequences on the telomeric and centromeric sides of the ABL gene. For example, Figure 9 provides a printout of the UCSC genome browser for the ABL gene that may be employed to identify suitable clone sequences to generate the ABL split apart probe pair. Preferably, repeat sequences are removed from both probe sets (See, e.g. Example 6), such that cell samples do not need to be blocked prior to in situ hybridization. Also, in preferred embodiments, the centromeric and/or telomeric probe sets (e.g. comprising fragments 0.1 to 8kb in length) have a combined hybridization length of at least 50kb, preferably 100kb, more preferably 200 kb, and most preferably at least 250 kb. In certain embodiments, the ABL.c (centromeric probe set) is approximately 250 kb in length (e.g. 200-300 kb), and the ABL.t (teleomeric probe set) is approximately 250 kb in length (e.g. 175-325 kb in length).

The ABL probe pair may be provided in a kit. For example, the kit comprises an ABL telomeric probe set, an ABL centromeric probe set, and instruction for employing the probe pair (e.g. to detect disease related to ABL rearrangement, such as those listed in Figure 4). The kits may comprise reagents necessary for performing FISH or CISH.

Interpreting the results of in situ hybridization on a cell sample (e.g. patient sample) may be performed as described above for the split apart probes of the present invention. For example, Figure 10 shows graphically how results look for "normal" (probe pairs next to each other) and translocation (one probe pair split apart). Figure 10 also shows the results that may be present in about 5-10% of CML cases that have a deletion that will, at least in some cases, result in loss of chromosomal material centromeric to the chromosome 9 breakpoint. ABL.c might be deleted in up to 5-10% of CML. The Zymed probe an advantage over the traditional bring-together probes to detect fusion genes, e.g. BCR/ABL probe from Vysis since the ABL split apart assay reveals both translocation and associated deletion.

In some embodiments, the patient sample (e.g. cell biopsy sample) is treated with the ABL probe pairs of the present invention, an ABL translocation is detected, and then the patient is identified as suitable for treatment with GLEEVACA, PD 17, or other suitable treatment. In other embodiments, the patient sample is treated with the ABL probe pairs of the present invention, ABL translocation is detected, and then the patient is administered GLEEVACA, PD17, or other suitable treatment.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: N (normal); M (molar); mM (millimolar); µM (micromolar); mol (moles); mmol (millimoles); µmol (micromoles); nmol (nanomoles); pmol (picomoles); g (grams); mg (milligrams); µg (micrograms); ng (nanograms); 1 or L (liters); ml (milliliters); µl (microliters); cm (centimeters); mm (millimeters); µm (micrometers); nm (nanometers); DS (dextran sulfate); and C (degrees Centigrade).

### EXAMPLE 1

### Generating HER2/neu Subtracted Probe Library

This example describes the generation of an exemplary HER2/neu subtracted probe library. This exemplary probe is useful in in-situ hybridization techniques such as CISH and FISH.

### 1 Selection of the BAC clone for detection of HER2 gene amplification.

Two BAC clones (312L7 and 359J8) were identified by PCR screening of a human BAC library (obtained from Research Genetics). These two BAC clones contain both the 5' and 3' ends ofHER-2 gene. The 5' and 3' ends of the two BAC clones were sequenced, and a BLAT search using these sequences was performed. In the UCSC Genome Browser December 22, 2001 Freeze, it was determined that BAC clone 3127L is located at 39829991-39946716 (117kb), and the BAC clone 359JB is located at 39890898-40010140 (119kb). The HER2 gene is located at 39915101-39943629. As such, both BAC clones contain the HER2 gene. FISH using each clone showed both of them bind specifically to the HER-2 gene locus on chromosome band 17q21 and absence of chimerism. The FISH signal generated using the two clones together was larger than that generated using either clone on its own.

### 2. Preparation of Tracer DNA.

The tracer DNA, which is used to generate a library of HER2 probe for ISH, was prepared by sonication of 10 ug of the purified BAC clone DNA to 0.1 - 8kb and size-fractionated on 1% agarose gel. The 0.5 -4 kb fractions were cut from the gel, purified using QIAquick gel extraction kit (QIAGEN, Santa Clarita, CA), blunt ended and ligated to the adapter. The T1/T2 adapter was constructed by annealing polyacrylamide gel electrophoresis (PAGE)-purified oligos T1 5'- CTG AGC GGA ATT CGT GAG ACC -3' (SEQ ID NO: 18) ("sense" oligo), and T2, 5'- PO4 GGT CTC ACG AAT TCC GCT CAG TT -3' (SEQ ID NO:19) ("antisense" oligo).

Adapter ligated fragments (100 ng) were then PCR amplified, in multiple 25 ml reactions, using the T1 sequence as primer. PCR cycling conditions were 94°C for 30 sec, 60°C for 30 second and 72°C for 3 min for 30 cycles, followed by 72°C for 10 minutes. Amplified fragments were size-fractioned (0.5 -4 kb fractions) on 1% agarose gel, then purified using QIAquick gel extraction kit.

### 3. Preparation of Biotin-labeled Driver DNA

Human high molecular weight Cot-1 (3mg) was blunt ended and ligated to the D40/D41 adapter constructed by annealing PAGE-purified oligos (SEQ ID NOS: 15 and 16 respectively). Adapter ligated fragments (100 ng) were then PCR amplified, in multiple 25 ul reactions, using 5'end biotin-labeled D40 (D40B, SEQ ID NO:17) sequence as primer. PCR cycling conditions were 94°C.for 30 sec, 60°C for 30 sec and 72°C for 3 min for 30 cycles, followed by 72°C for 10 min. The PCR product was purified by phenol:chloroform:isoamyl. The pellet was dried and drive DNA carefully re-dissolved at 1.5-2.5 mg/ml in BE buffer (10 mmol/L 2hydroxyethyl] piperazine-N'- 3-propanesulfonic acid (NaEPPS),1 1 mmol/L EDTA, pH 8.0).

### 4. Subtraction Hybridization

Genomic subtractive hybridization removed sequences from a tracer DNA population by hybridizing with a molar excess of driver DNA. The driver DNA is chemically modified, with a biotin, such that it may be selectively removed from solution along with driver-tracer hybrid molecules. Briefly, HER2 tracer DNA was repeatedly hybridized with 40-fold excess of biotin-labeled Driver DNA containing the repetitive sequences (Alu and LINE elements). Consequently, repetitive sequences presenting the HER2 region were quantitatively removed. The detailed methods are set forth below.

Subtraction was performed by mixing 250 ng of tracer DNA with 10 mg of biotin-labeled driver DNA, 2 mg of T1, 5 mg of yeast tRNA as carrier. This mixture was denatured at 100°C for 2 min, lyophilized, re-dissolved in 5 ml of EE buffer/1 mol/L NaCl, then incubated at 65°C for 24 to 48 hours. Biotinylated molecules (including tracer-driver hybrids) were removed using avidin-polystyrene beads. Remaining unbiotiylated tracer fragments were precipitated in ethonal before proceeding with the next round of subtraction. Each of three rounds of subtraction was performed as described above. After the third round, remaining tracer fragments were amplified by PCR using the T1 sequence as primer.

### 5. Probe Library Preparation

After three rounds of subtraction and 3 rounds of PCR, the HER2 DNA probe library was labeled with digoxigenin (DIG) using random octamer primer kit (Gibco BRL/Life Technologies). The final nucleotide concentrations for DIG labeling were 0.2 mmol/L dCTP, dGTP, dATP, 0.13 mmol/L dTTP, and 0.07 mmol/L Dig-11-dUTP (Boehringer Mannheim/Roche). Residual primers and unincorporated nucleotides were removed by S-200HR spin column chromatography (Amersham Pharmacia Biotech Inc.). The purified products were precipitated in ethanol and dissolved in a solution containing 50% formamide, 10% dextran sulfate, and 2x SSC (0.3 mol/L sodium chloride, 0.03 mol/L sodium citrate, pH 7.0).

### EXAMPLE 2

### Chromogenic In-Situ Hybridization Detection of HER2/neu

This example describes performing chromogenic in-situ hybridization with the HER2/neu probe library generated in Example 1, as well as general procedures for evaluating CISH results. CISH was done on 4 µm-thick tissue sections mounted on Superfrost/plus microscope slides (Fisher, Pittsburgh, PA). The slides were baked 2-4 hours at 65°C and then deparaffinized 10 minutes in Xylene (2 times) and 5 minutes in ethanol (3 times). Air-dried tissue sections were placed in a plastic Coplin jar containing the CISH Pretreatment Buffer (0.1M Tris/0.05 M EDTA, pH 7.0, SPOT-Light Tissue Pretreatment Kit, Zymed), and loosely capped. They were heated at 199°F for 15 min in the microwave with a temperature probe (GE Profile Sensor convection). The temperature probe was placed in a separate plastic Coplin jar without a cap. As a result of capping the Coplin jar, the tissue sections reached a boiling temperature (100 degrees Celsius), which was evidenced by the solution in the jar boiling when the microwave was stopped and the jar examined.

The slides were washed immediately with deionized water after heat pretreatment. Enzyme digestion was followed by covering the section with prewarmed 37°C pepsin (0.0625% pepsin, pH 2.3, SPOT-Light Tissue Pretreatment Kit, Zymed) and by incubating at 37°C for 3±1 minutes. The slides were then washed with deionized water, dehydrated with graded ethanol, and air-dried. The ready-to-use DIG-labeled HER2 probe (See, Example 1) or biotin-labeled chromosome 17 centromeric probe (SPOT-LIGHT Chromosome 17 Centromeric Probe, Zymed Laboratories, Inc.) was applied to the center of the coverslip. The coverslip was placed with probe side down on the tissue sample. 15 µl or 20µl of the probe was used for 22 x 22 mm or 24 x 32 mm coverslips according to the size of the tissue sections to be covered. After sealing the edges of the coverslips with rubber cement, the tissue sections and the probes were denatured at 94 °C for 5 minutes by placing the slides in the slide block of the PCR machine (MJ research, Watertown, MA). Hybridization was done in the same slide block at 37°C overnight The stringent wash was done with 0.5 x standard saline citrate at 75-80°C for 5 minutes.

Next, the endogenous peroxidase activities were blocked in 3% H₂O₂ diluted with methanol for 10 minutes. The unspecific staining was blocked by applying the Cas-Block^{™} (0.25% casein, 0.2% gelatin, and 10mM PBS, pH 7.4) on the tissue section and by incubating for 10 minutes. After blotting off the Cas-Block^{™}, FITC conjugated mouse anti-DIG antibody was applied on the tissue section and incubated for 45 minutes at room temperature. After three times washing, each 2 minutes with PBS and Tween 20, HRP conjugated sheep anti-FITC antibody was applied on the tissue section and incubated for 45 minutes at room temperature, followed by DAB development for 30 minutes. The biotin-labeled chromosome 17 centromere probe was detected with sequential incubation with HRP conjugated streptavidin for 45 minutes at room temperature and DAB development (CISH Centomere Detection Kit, Zymed) for 30 minutes. Tissue sections were counterstained with hematoxylin, dehydrated, and coverslipped. Positive controls were included in each staining run.

Evaluation of CISH results. CISH results were evaluated using a bright field microscope (Nikon, E400) equipped with 10x, 20x, and 40x dry objectives with 10x oculars (see Table 4). For evaluation of HER2 CISH results please see Table 5. An individual HER2 gene or chromosome 17 centromere signal appears as a small, single dot. Targeted HER2 gene amplification is typically seen as large DAB-stained clusters or many dots in the nucleus.

**TABLE 4: Signal Visualization**

| Magnification | CISH Signal |
|---|---|
| 10x | Individual signals are barely visible and may be missed. |
| 20x | Individual signals are small but clearly discernible. |
| 40x | Individual signals are easily identified |
| 60x or 100x | Not necessary |

**Table 5: Exemplary Criteria of HER2 gene status by CISH**

| | |
|---|---|
| Amplification | >10 copies or large clusters of HER2 gene (amplicon) per nucleus in >50% of cancer cells. |
| Low Amplification | 6-10 copies of HER2 gene or small cluster of HER2 gene (amplicon) per nucleus in >50% of cancer cells. |
| | Biotin-labeled Spot-Light chromosome 17 centromeric probe may be applied for CISH to confirm that 6-10 copies of HER2 gene (<5% cases) were due to HER2 gene amplification but not chromosome 17 polysomy. |
| No Amplification | 1-5 copies of HER2 gene per nucleus in >50% of cancer cells. |
| | 3-5 copies of HER2 gene per nucleus is due to chromosome 17 polysomy. There is no need for chromosome 17 centromeric CISH. Occasionally, it is found that HER2 has 3-5 copies and chromosome 17 centromere has 1-2 copies in >50% of cancer cells (HER2/chr.17cen ratio is ≥2), it is due to what sometimes was seen by CGH of duplication of chromosome arm 17q. |

The CISH staining results are clearly seen using a 40x objective in tissue sections which are counterstained with, for example, hematoxylin. An individual gene or chromosome centromere signal appears as a small, single dot. Targeted gene amplification is typically seen as large DAB-stained clusters or many dots in the nucleus or mixed clusters and multiple dots (>6 dots per nucleus). Tumors with no targeted gene amplification show typically 1 to 5 dots per nucleus. 3-5 dots per nucleus in more than 50% of tumor cells are due to chromosome polysomy.

### EXAMPLE 3

### Exemplary TopoIIα Probe and Other TopoIIα Probes

This Example describes an Exemplary TopoIIα probe useful for detecting TopoIIα copy number in, for example, FFPE tissue sections, fresh tissue sections, cell preparations, and metaphase chromosome spreads using *in situ* hybridization detection methods such as FISH and CISH. This Example also describes procedures for constructing similar probes.

The Exemplary TopoII*α* probe described in this Example is available from Zymed Laboratories (South San Francisco, CA, Cat. No. 84-0600) as a library of fragments ranging in size from about 0.5 to 4 kb in size. The nucleic acid sequence of the Exemplary TopoIIα probe is an approximately 170 kb sequence from human chromosome seventeen (17) that encompasses the TopoIIα gene, but does not contain the HER2/*neu* gene. FISH experiments revealed that the probe binds specifically to the topoIIα gene locus on chromosome band 17q11-21 and absence of chimerism. PCR with HER2/*neu* specific primers demonstrated that the sequence of the Exemplary TopoIIα probe does not contain the HER2/*neu* gene.

### i) Selection of PAC clone for detection of TopoIIα gene amplification

In order to isolate the PAC clone for topoIIα, PAC clones probes for *topoIIα* were obtained by PCR-based screening of a PAC library. A chromosome 17 pericentromeric probe (p 17H8) was used as a reference probe to determine the overall copy number of chromosome 17. The specificity of the *topoIIα* probe was confirmed by PCR with *topoII*α specific primers. This *topoIIα* probe does not contain HER-2 DNA sequence since there is no amplification using 3 pairs of HER-2 specific primers covering 5' end, middle, and 3' end of HER-2. The PCR-analysis showed that the *topoIIα* probe did not recognize sequences from HER- *2*/*neu.*

Sequencing the ends of the Exemplary probe revealed that this sequence is bounded on the 3' end by the sequence shown in Figure 1A (SEQ ID NO:9), and bounded on the 5' end by the sequence shown in Figure 1B (SEQ ID NO:10). Comparison with the published human genome sequence in chromosome 17q 11-21 region in Gene Bank revealed that the sequence of the Exemplary probe is located about 500 kb downstream of the HER2/*neu* gene.

The sequence of the Exemplary probe may be constructed, for example, by employing the 3' and/or 5' ends of the Exemplary probe sequence (*i.e.* SEQ ID NOS:9 and. 10). For example, these sequences may be used to screen a library of human sequences, such that a clone containing this sequence is found and isolated. This clone can be further manipulated by standard molecular biology techniques such that sequences similar to, or identical to, the Exemplary probe sequence are generated. SEQ ID NOs:9 and 10 may also be employed to screen human gene sequence databases (e.g. at chromosome 17) such that the sequences between SEQ ID NOs:9 and 10, and near SEQ ID NOs:9 and 10, may be determined (and then used to generate sequences that are the same or similar to the Exemplary probe sequence using standard molecular biology techniques). Preferably, if sequences similar to the Exemplary probe sequence are generated, the length of the resulting sequence is selected such that it is between 100 kb and 1 megabase (total length of the library of fragments that make up the probe) and is capable of hybridizing to human chromosome 17 (*e.g.,* at a region that contains the TopoII*α* gene and not the HER2/*neu* gene).

To confirm that the Exemplary probe contained the TopoII*α* gene sequence, a PCR test was conducted. In particular, the Exemplary probe sequence was used as a template and Two topoIIα primers were used (TopoIIαA: 5-'GCC TCC CTA ACC TGA TTG GTTA-3', SEQ ID NO:11; and TopoIIαB: 5'-CTC AAG AAC CCT GAA AGC GACT-3', SEQ ID NO:12). The PCR reaction was performed in a volume of 25 ul containing 100 ng of Tracer DNA, 20 pmols of each primer, 1 x KlenTaq DNA polymerase (Clonetech), and 200 uM of each dNTPs (Roche). The PCR was performed for 30 cycles of 94 degrees Celsius for 1 minute. The resulting gel revealed a clear TopoIIα PCR product (259 bases). This same type of PCR test may be used on other TopoIIα probe sequences that are generated to confirm that the TopoII*α* gene is encompassed by the probe.

### ii) Preparation of Tracer DNA

The Exemplary topoIIα probe (or "tracer DNS) may be used to generate a library of fragments with a total length of 100kb to 1 megabase (e.g. 170 kb total length), with the repetitive sequences substantially removed from this library as described below. In this example, the trace DNA, was prepared by sonication of 30 ug of purified PAC clone DNA to 0.1- 8kb and size-fractionated on 1 % agarose gel. The 0.5 - 4 kb fractions were cut from the gel, purified using QIAquick gel extraction kit (QIAGEN, Santa Clarita, CA), blunt ended and ligated to the TopoIIα1/ TopoIIα2 adapter. The TopoIIα1/ TopoIIα2 adapter was constructed by annealing polyacrylamide gel electrophoresis (PAGE)-purified oligos TopoIIα 1 5'-(PO4) GCT ACG GTC TGC TCA GGA CAG TT -3' ("antisense" oligo, SEQ ID NO: 13), and TopoIIα 2 3'-CGA TGC CAT ACG ACT CCT GTC - 5' ("sense" oligo, SEQ ID NO:14). Adapter ligated fragments (100 ng) were then PCR amplified, in multiple 25 ml reactions, using the TopoIIα2 sequence as primer. PCR cycling conditions were 94°C for 30 sec, 60°C for 30 sec and 72°C for 3 min for 30 cycles, followed by 72°C for 10 min. Amplified fragments were size-fractioned (0.5 -4 kb fractions) on 1% agarose gel, then purified using QIAquick gel extraction kit.

### iii) Preparation of Biotin-labeled Driver DNA

Fragments of high molecular weight Cot-1 (3mg) ranging in size from 0.4 and 2 kb were gel purified, blunt ended, and ligated to a D-40/D-41 adapter constructed by annealing PAGE-purified oligos 5'AATTCTTGCGCCTTAAACCAAC (D-40) SEQ.ID. NO: 15 and 5'GTTGGTTTAAGGCGCAAG (D-41) SEQ. ID. NO: 16. Adapter ligated fragments (100 ng) were then PCR amplified, in multiple 25 ml reactions, using 5'end biotin-labeled D40 (5'.(biotin) AATTCTTGCGCCTTAAACCAAC (D-40B) SEQ. ID. NO:17) sequence as primer. PCR cycling conditions were 94°C for 30 sec, 60°C for 30 sec and 72°C for 3 min for 30 cycles, followed by 72°C for 10 min. The PCR product was purified by phenol:chloroform:isoamyl. The pellet was dried and drive DNA carefully re-dissolved at 1.5-2.5 mg/ml in EE buffer (10 mmol/L 2hydroxyethyl] piperazine-N'-3-propanesulfonic acid (NaEPPS), 1 mmol/L EDTA, pH 8.0).

### iv) Subtraction Hybridization

Genomic subtractive hybridization removed sequences from a tracer DNA population by hybridizing with a molar excess of driver DNA. The driver DNA is chemically modified, e.g. with a biotin, such that it may be selectively removed from solution along with driver-tracer hybrid molecules. Briefly, TopoIIα tracer DNA was repeatedly hybridized with 40-fold excess of biotin-labeled Driver DNA containing the repetitive sequences (Alu and LINE elements). Consequently, repetitive sequences present in the TopoIIα region were quantitatively removed. The detailed methods are set forth below.

Subtraction was performed by mixing 250 ng of tracer DNA with 10 mg of biotin-labeled driver DNA, 2 mg of TopoIIα2, 5 mg of yeast tRNA as carrier. This mixture was denatured at 100°C for 2 min, lyophilized, redissolved in 5 ml of EE buffer/1 mol/L NaCl, then incubated at 65°C for 24 to 48 hours. Biotinylated molecules (including tracer-driver hybrids) were removed using avidin-polystyrene beads as described. Remaining unbiotiylated tracer fragments were precipitated in ethonal before proceeding with next round of subtraction. Each of three rounds of subtraction was performed as described above. The subtraction process resulted in at least 95% of the repetitive sequences being removed from the probe library. After the third round, remaining tracer fragments were amplified by PCR using the TopoIIα2 sequence as primer.

### v). Subtracted Nucleic Acid Probe Library Final Preparation

After three rounds of subtraction and 3 rounds of PCR, the TopoIIα subtracted nucleic acid probe library was labeled with digoxigenin (DIG) using the random octamer primer kit (Gibco BRL/Life Technologies). The final nucleotide concentrations for DIG labeling were 0.2 mmol/L dCTP, dGTP, dATP, 0.13 mmol/L dTTP, and 0.07 mmol/L Dig-11-dUTP (Boehringer Mannheim/Roche)/ Residual primers and unincorporated nucleotides were removed by S-200HR spin column chromatography (Amersham Pharmacia Biotech Inc.). The purified products were precipitated in ethanol and dissolved in a solution containing 50% formamide, 10% dextran sulfate, and 2x SSC (0.3 mol/L sodium chloride, 0.03 mol/L sodium citrate, pH 7.0).

While the Exemplary probe topoII*α* library is labeled with digoxigenin (DIG). The Exemplary probe sequence, or other probes with the same or similar sequences, can be labeled with any type of detectable label (*e.g.,* such that the probe can be detected during *in situ* hybridization procedures such as FISH or CISH). Also, the Exemplary probe's specificity has been demonstrated by CISH detection methods (data not shown) on the mammary gland adenocarcinoma MCF-7 (ATCC# HTB-22) which does not have TopoIIα gene amplification or deletion, and mammary gland adenocarcinoma cells MDA-MB-361 cell (ATCC# HTB-27), which has the TopoIIα gene deleted.

### EXAMPLE 4

### In Situ Hybridization Methods with the Exemplary TopoIIα Probe

This example describes *in situ* hybridization methods (CISH and FISH) that may be used with TopoIIα probes, such as the Exemplary TopoIIα Probe described in Example 2. In particular, this Example describes *in situ* hybridization methods with the Exemplary probe in Formalin-Fixed, Paraffin-Embedded (FFPE) Tissue Samples, as well as Cell Sample/Metaphase Chromosome samples. Finally, this example describes a quality control procedure that may be used with any of these methods.

### A. Single-Color CISH For Detection of DIG labeled Exemplary TopoIIα probe on FFPE Tissue Sections

### I. PRETREATMENT

1. Deparaffinization

| | |
|---|---|
| Xylene | 10 Min x 2 |
| 100% EtOH | 5 Min x 3 |
| Air dry slides | |

2. Heat treatment
(boil the slide by using microwave with temperature probe, or pressure cooker or hot plate)

| | |
|---|---|
| Tris-EDTA buffer, pH 7.0 | 15 Min, 96-100°C |
| (SPOT-Light Tissue Heat Pretreatment Buffer, Cat.# 00-8401) | |
| dH₂O | 2 Min x 3 |

3. Pepsin digestion:

| | |
|---|---|
| Pepsin at 37°C | 3 Min. |

Note: different concentrations of pepsin and incubation times (1-10 min) may be required depending on tissue fixation and type of tissue. Excessive digestion will cause loss of nuclei and chromosome structure, while inadequate digestion may result in loss of signal.

| | |
|---|---|
| dH₂O | 2min x 3 |

4. Dehydration with graded alcohol

| | |
|---|---|
| 70% EtOH | 2 min |
| 85% EtOH | 2 min |
| 95% EtOH | 2 min |
| 100% EtOH | 2 min |
| 100% EtOH | 2 min |

5. Air dry slides
6. Label slides with pencil

### II. Option 1: CO-DENATURATION and HYBRIDIZATION: (use PCR machine with slide block, or heating block with temperature digital display and humidity slide chamber and 37°C incubator)

1. Add probe: add 12-15 ul of probe to the center of 22 x 22 mm coverslip, or 20 ul of probe to the center of 24 x 32 mm coverslip.
2. Coverslip: coverslip slide at appropriate tissue sample area.
3. Seal with rubber cement: seal edges of coverslip with thin layer of rubber cement for preventing evaporation during incubation.
4. Denaturation at 94°C for 5 min: place the slides in a slide block of PCR machine, or on a heating block with temperature digital display.
5. Incubation at 37°C overnight: leave the slides in the slide block of PCR machine or place the slides in a dark humid box in a incubator.

### Option 2: SEPARATE DENATURATION (e.g., when PCR machine or heating block are not available)

1. Denature tissue in fresh made denaturing buffer at 75°C 5 min.
Denaturing buffer: 4 ml 20 x SSC (20x SSC buffer = 0.3M Sodium Citrate, with 3M NaCl, ph 7.0), 8 ml ddH₂O, 28 ml formamide.
(For more than one slide samples, add 1 °C per slide. For example, if 2 slides are used, set temperature to 76°C).
2. Dehydration with graded alcohol

| | |
|---|---|
| 70% EtOH | 2 min, at-20°C |
| 85% EtOH | 2 min, at -20°C |
| 95% EtOH | 2 min, at -20°C |
| 100% EtOH | 2 min, at RT |
| 100% EtOH | 2 min, at RT |

3. Air dry slides. At the same time process step 4.
4. Denature labeled probe 75°C, S min.
5. Place denatured probe in ice immediately.
6. Add probe: add 12-15 ul of denatured probe to the center of 22x22 mm coverslip.
7. Coverslip slides at appropriate tissue sample area.
8. Incubation: place slides in a dark humid box at 37°C for overnight (more than 14 hours).

### III. STRINGENCY WASH:

1. After hybridization, carefully remove rubber cement and coverslip.
2. Stringency wash: Wash slides in 0.5xSSC at 75°C for 5 min.
   (Add 1 °C per slide for more than 2 slides, but do not go higher than 80°C)
3. dH₂O wash: 2 min x 3

### IV. IMMUNODETECTION:

1. 3% H₂O₂ in absolute Methanol: (for Peroxidase Quenching) 10 min
2. 1 x PBS (10mM/Tween 20 (0.025%) wash: 2 min x 3
3. Add blocking reagent 2 drops/slide at RT (CAS-Block; 0.25% casein, 0.2% gelatin, and 10mM PBS, pH 7.4) 10 min
   Note: use enough reagents to cover all the area of tissue.
4. Blot off blocking reagent, DO NOT RINSE.
5. Add FITC-anti-dig antibody 2 drops/slide at RT 45 (30-60) min
   Note: use enough reagents to cover all the area of tissue.
6. 1 x PBS/Tween 20 (0.025%) wash 2 min x 3
   If FISH is desired, add 1 drop of VECTASHIELD Mounting Medium with DAPI (Vector, Cat. No. H-1200) on the section, then coverslip. Incubate for 10 min at RT in a dark chamber box before performing fluorescent microscopy. After analysis is done, remove coverslip, wash slide in 1 x PBS/Tween 20 (0.025%) 3 times, each time 2 min. Continue to next step.
7. Add HRP-anti-FITC 2drops/slide at RT 45 (30-60) min
   Note: use enough reagents to cover all the area of tissue.
8. PBS/Tween (0.025%) wash 2 min x 3
9. Add DAB, 3 drops/slide, 30 min
   Note: use enough reagents to cover all the area of tissue.
   (Make DAB signal by adding 1 drop of each reagent A (CAS-BLOCK), B (FITC-Sheep anti-Digoxigenin) and C (HRP-Goat anti-FITC) to 1ml dH₂O, then mix well)
10. Wash with running tap water. 2 min.

### V. COUNTERSTAINING AND COVERSLIPPING

1. Counterstain with hematoxylin 6 sec -1 min.
   Time of counterstaining is dependent on tissues used. Dark counterstaining is not recommended as it may obscure the positive signal.
2. Wash with running tap water 2 min
3. Dehydrate with graded EtOH
   (70%, 85%, 95%,100%, 100%) 2 min each
4. Xylene 2 min x 2
5. Coverslip with Histomount (Cytoseal 6.0, cat. # 8310-16, Stephen Scientific).

### V. MICROSCOPY

Visualize probe in cells with a bright field microscope.

### B. Cell Sample or Metaphase Chromosome Sample

Fix cell sample on HISTOGRIP or Superfrost/Plus coated (or other) glass slide.

### Pretreatment

1. Immerse slides in 2x SSC buffer (20x SSC buffer = 0.3M Sodium Citrate, with 3M NaCl, ph 7.0) at 37 degrees Celsius for 60 minutes.
2. (Optional) Pretreat cells with SPOT LIGHT Cell Pretreatment Reagent (or other Pepsin composition in acidic buffer) for 5 minutes at 37 degrees Celsius. Incubation time may be from 1-10 minutes depending on cell type and slide-making conditions. Excessive pepsin digestion will cause loss of nuclei and chromosome structure. Inadequate digestion may result in loss of signal.
3. Wash in dH₂0 for 3 x 2 minutes at room temperature (RT).
4. (Optional) Immerse slides in 10% buffered formalin for 1 minute at RT.
5. Wash in dH₂0 for 3 x 2 minutes at RT.
6. Dehydrate slides in 70%, 85%, 95%, and 100% ethanol for 2 minutes each, and then air dry.
   Slides are now ready for ISH procedure (alternatively, slides can be stored in 70% ethanol at -20 degrees Celsius.

### Denaturation and Hybridization

1. Add 15 ul of Exemplary topoIIα probe (probe) to the center of the sample and cover with a 22 x 22 mm coverslip (use more probe for bigger sample and larger coverslip).
2. Seal edges of coverslip with thin layer of rubber cement to prevent evaporation of probe solution during incubation.
3. Denature the slides on a hot plate or slide warmer at 80 degrees Celsius for 3 minutes (2-5 minute range), or in the slide block of a PCR thermal cycler.
4. Place slide in a dark humidity box or in the slide block of a PCR thermal cycler for 16-24 hours at 37 degrees Celsius.

### Stringency Wash

1. Remover rubber cement and coverslip.
2. Immerse slides in 0.5x SCC buffer, using a Coplinjar, for 5 minutes at 72 degrees Celsius (note - this temperature is based on one slide, but each slide causes a 1 degree Celsius drop in solution temperature. Therefore, if there is more than one slide, adjust the water bath temperature accordingly. For example, if washing 4 slides, adjust the water bath temperature to 75 degrees Celsius. Do not go higher than 80 degrees Celsius.).
3. Wash slides in PBS/Tween 20 buffer (1 part Tween-20, 3900 parts 0.1 M PBS) for 3 x 2 minutes at RT.
   Perform Immunodetection and Counterstaining-Coverslipping as described above in part A above.

### C. Quality Control Procedures

Quality control over the accuracy of the above procedures may be assured by using some or all of the controls described below.

Positive (Amplification) Tissue Control: External positive control materials for clinical research should be fresh autopsy/biopsy/surgical specimens fixed, processed, and embedded as soon as possible in the same manner as the patient sample(s). Specimens processed differently from the specimen sample(s) validate reagent performance, and do not verify tissue preparation. Positive tissue controls are indicative of correctly prepared tissues and proper staining techniques. One positive tissue control for each set of test conditions should be included in each run.

Tissues used for the positive control materials should be selected from specimens with well-characterized levels of TopoIIα gene. Approximately 5-10% of breast cancer tissue has TopoIIα gene amplification and may be a useful source of positive control tissue.

Known positive controls should be utilized for monitoring the correct performance of processed tissues and test reagents, rather than as an aid in interpreting sample results. If the positive tissue controls fail to demonstrate positive staining, results with the specimen samples should be considered invalid.

**Negative or Normal (Diploid) Tissue Control:** Human diploid tissue samples normally have two TopoIIα gene copies in each cell. Therefore, a true negative tissue sample is not available. However, normal tissue can be used as a negative control for gene amplification or deletion. Use a negative tissue control (known to be diploid) fixed, processed, and embedded in the same manner as the sample(s) with each staining run. This will verify the specificity of the ISH probe, and provide an indication of non-specific background staining (false positive staining).

A negative tissue control that is separate from the sample is known as an 'external' negative control. If an external negative tissue control is not available then a normal section of the sample can serve as an 'internal' negative tissue control.

**Reagent (No-Probe) Control**: A reagent control is run on a section of sample specimen without the probe. The reagent control is useful in evaluating the possibility of nonspecific staining, particularly when performing ISH in tissue sections. The reagent control should be stained in the same way as the test samples except that hybridization buffer, that does not contain the probe, should be used during the hybridization step. Slide pretreatment, denaturation, and immunodetection should be performed under the same conditions as test samples.

### EXAMPLE 5

### Generation of EGFR Subtracted Probe Library

This example describes the generation of an EGFR subtracted probe library. This probe library was generated substantially as described in Example 1, except where otherwise specified.
1) Selection of the BAC and PAC clone for detection of EGFR gene amplification.
   Two BAC clones (343B1 and 339F13), one PAC clone (1091E12) were identified by human genome project (obtained from Research Genetics). These three clones were confirmed to contain EGFR gene by PCR, and FISH using each clone showed all of them bind specifically to the EGFR gene locus on chromosome band 7p12 with absence of chimerism. A BLAT search of the UCSC genome browser August 6, 2001 freeze indicated that the three clones overlapped, and combined to span a 303 kb distance from 59711021-60014071, that encompassed the EGFR gene.
2). Preparation of Tracer DNA.
   The PAC and BAC clones were manipulated in the same manner as described in Example 1 above. The primers for adapter used for the EGFR probe library are as follows:
   *EGFR.A 5'-(PO4) ACC GTA GGA CTC TGC TGG CGA TT-3' (SEQ ID NO:20) ("antisense" oligo), and*
   *EGFR.B 5'-TCG CCA GCA GAG TCC TAC GGT-3' (SEQ ID NO. 21) ("sense" " oligo)*
3). Preparation of Biotin-labeled Driver DNA
   Same as Example 1.
4). Subtraction Hybridization
   Same as HER2 probe (Example 1) except T1 primer was replaced by EGFR B primer.
5). Probe Preparation
   Same as HER2 probe (See, Example 1).

### EXAMPLE 6

### ABL Subtracted Split-Apart Probe Pair

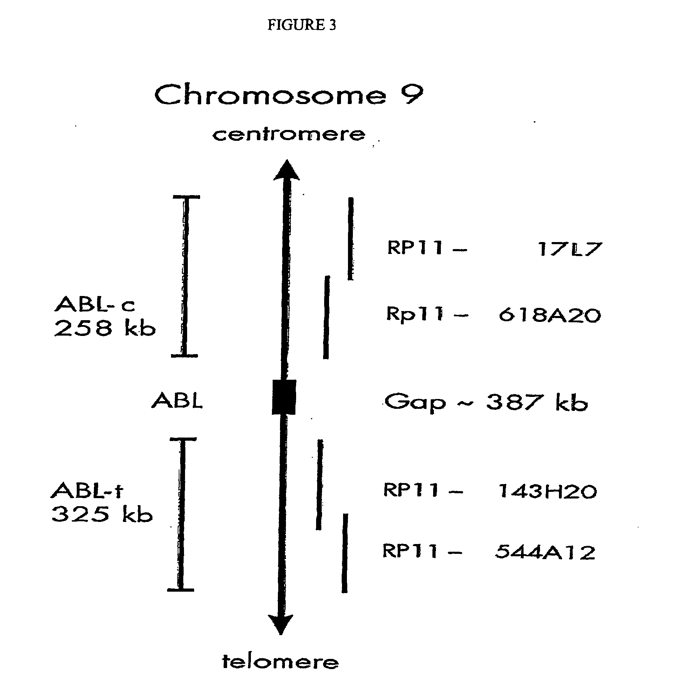

This example describes the generation of an ABL split-apart subtracted probe pair library. This probe library was generated substantially as described in Example 1, except where otherwise specified. Importantly, this probe pair is designed to detect chromosome translocations in a unique "split-apart" manner. Conventional BCR/ABL probe pairs are located on different chromosomes in the "normal" state (e.g. non-cancerous), and are only located side by side when translocation occurs. The spit-apart ABL probes of the present example are designed such that that pair is located on the same chromosome in the "normal" state (e.g. non-cancerous), and are only located on separate chromosomes when translocation has occurred in the sample being tested.
1) Selection of the BAC clones for detection of BCR/ABL translocation.
   Two BAC clones, RP11-618A20 (accession No. AL354898.10) and RP11-17L7 (accession No. AL353695.7) were used to generate the ABL.c (centromeric side) probe (about 258 kb in size.). Figure 9 shows the UCSC genome browser for the ABL gene, which may be used to selected additional or alternate clones that may be used to generate the ABL.c and ABL.t probes of the present invention. Also,two BAC clones, RP11-143H20 (accession No. AL355872.13) and RP11-544A12 (accession No. AL157938.22) were used to generate the ABL.t (telomeric side) probe (about 250 kb in size). FISH using these clone showed all of them bind specifically to chromosome band 9q34 with an absence of chimerism.
2). Preparation of Tracer DNA.
   The BAC clones were manipulated in the same manner as described in Example 1 above. The primers for adapter:
   *ABL.cA* 5*-(PO₄) ATC GGT GTA GCC TGA ATG GAC TT-3'* (SEQ ID NO:22)
   *ABL. cS5'- GTC CAT TCA GGC TAC ACC GAT-3'* (SEQ ID NO:23)
   *ABL.tA5'-(PO₄) CAT CAT TCG GTC AGA GGC A CT TT-3'* (SEQ ID NO:24)
   *ABL.tS5'- AGT GCC TCT GAC CGA ATG ATG-3'* (SEQ ID NO:25)
3). Preparation of Biotin-labeled Driver DNA
   Same as Example 1.
4). Subtraction Hybridization
   Same as HER2 probe (Example 1) except T1 primer was replaced by ABL.cS and ABL.tS primers for ABL.c and ABL.t probe respectively.
5). Probe Preparation
   Same as HER2 probe (See, Example 1). The probe pair (centromeric and telemoric probes) give about a 109 kb gap on the centromeric side and a 104 kb gap on the telomeric side of the ABL gene (See Figure 3 and 4). This labeled probe pair, for example, may be used to detect ABL translocations (e.g. BCR-ABL translocation) by in situ hybridization techniques (e.g. FISH and CISH). For example, translocations in the ABL gene are found in CML, acute lymphoblastic leukemai (ALL) acute non-lymphocytic leukemia (ANLL), and acute myeloid leukemia (AML) (See, section VI above). In addition these probes can detect variant ABL translocations, such as TEL-ABL, which are found in other types of leukemia.

### EXAMPLE 7

### Generation of N-MYC Subtracted Probe Library

This example describes the generation of an N-MYC subtracted probe library. This probe library was generated substantially as described in Example 1, except where otherwise specified.
1) Selection of the BAC clone for detection of N-Myc gene amplification.
   Two BAC clones (2014F22 and 2121A13339F13) were identified by Caltech OncoBAC screening effort and human genome project (obtained from Research Genetics). These two clones were confirmed to contain N-Myc gene by PCR FISH using each clone showed all of them bind specifically to the N-MYC gene locus on chromosome band 2p24.3 with an absence of chimerism. The FISH signal generated using the two clones together was larger than that generated using either clone on its own.
2). Preparation of Tracer DNA.
   The BAC clones were manipulated in the same manner as described in Example 1 above. The primers for adapter:
   *AF3S 5'-TCT TCA CGA CAC GACAGC CAG -3'* (SEQ ID NO:26) *("sense" oligo)*
   *AF3α 3'-TTAGA AGT GCT GTG CTG TCG GTC (PO₄)-5'* (SEQ ID NO:27) *("antisense" oligo)*
3). Preparation of Biotin-labeled Driver DNA
   Same as Example 1.
4). Subtraction Hybridization
   Same as HER2 probe (Example 1) except T1 primer was replaced by AF3S primer.
5). Probe Preparation
   Same as HER2 probe (Example 1).

### EXAMPLE 8

### Generation of SYT Subtracted Probe Pair Library

This example describes the generation of an SYT (Synovial Sarcoma) subtracted probe pair library. This probe library was generated substantially as described in Example 1, except where otherwise specified. Importantly, this probe pair is designed to detect chromosome translocations in a unique "split-apart" manner. Conventional SYT probe pairs are located on different chromosomes in the "normal" state (e.g. non-cancerous), and are only located side by side when translocation occurs. The spit-apart SYT probes of the present example are designed such that that pair is located on the same chromosome in the "normal" state (e.g. non-cancerous), and are only located on separate chromosomes when translocation has occurred in the sample being tested.
1. Selection of the BAC clones for detection of SYT translocation.
   Clones from both sides of the SYT (SS18) gene are employed to generate a split-apart subtracted probe pair library. For example, one or more of the following clones may be used to generate the SYT.c (centromere side probe): RP11-885J19 (See, accession No. AP001326); RP11-689N18 (See accession No. AP001121); RP11-5F22 (See, accession No. AC011268); RP11-326M20 (See accession No. AC019306); RP11-540M4 (See, accession No. AC007768). Preferably the first four clones listed are employed, and optionally the fifth clone is added. In order to generate the SYT.t (telomeric side probe) one or both of the following clones may be employed; RP11-802C10 (accession No. AP002752), and RP11-774F2 (accession No. AP001451).
2. Preparation of Tracer DNA.
   The clones were manipulated in the same manner as described in Example 1 above. The primers for adapter:
   *SYT.c1 5'-ATG CGT CCA CCT TGA CCYTAC-3'(SEQ* ID NO:28)
   *SYT.c2 5'=(PO₄) GTA AGG -TCA AGG TGG ACG CAT-TT-3'* (SEQ ID NO:29)
   *SYT.tS 5' ATA GCC CCG AAT CAG GTG GAA-3'* (SEQ ID NO:30)
   *SYT.tA 5'-(PO₄)-TTC CAC CTG ATT CGG GGC TAT TT-3'* (SEQ ID NO:31)
3. Preparation of Biotin-labeled Driver DNA
   Same as Example 1.
4. Subtraction Hybridization
   Same as HER2 probe (Example 1) except T1 primer was replaced by SYT.c1 and SYT.tS primers for SYT.c and SYT.t probe respectively.
5. Probe Preparation
   Same as HER2 probe (Example 1).

Various modifications and variations of the described method and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in medicine, immunology, chemistry, and molecular biology or related fields are intended to be within the scope of the following claims.

## Claims

1. A method for performing chromogenic in-situ hybridization, comprising:
a) preheating a biological sample in a pretreatment buffer at a temperature of a least 96 degrees Celsius,
b) exposing said biological sample to an enzyme digestion solution,
c) contacting said biological sample with a subtracted probe library under conditions such that said subtracted probe library hybridizes to a target region in said biological sample,
d) adding a detection molecule linked to an enzyme to said biological sample under conditions such that said detection molecule binds: i) to said labeled subtracted probe library, or ii) an intermediate molecule linked to said subtracted probe library, and
e) adding a colorimetric substrate to said biological sample.

2. The method of claim 1, further comprising step f) detecting said target region.

3. The method of Claim 2, wherein said detecting comprising visualizing said colorimetric substrate with a microscope.

4. The method of Claim 3, wherein said microscope is a bright-field microscope.

5. The method of any of claims 1 to 4, wherein said subtracted probe library is configured for detecting HER2 gene amplification.

6. The method of any of claims 1 to 4, wherein said subtracted probe library is configured for detecting topoIIα gene amplification.

7. The method of any of claims 1 to 4, wherein said subtracted probe library is configured for detecting EGFR gene amplification.

8. The method of any of claims 1 to 4, wherein said subtracted probe library is configured for detecting N-MYC gene amplification.

9. The method of any preceding claim, wherein said subtracted probe library comprises a probe pair library.

10. The method of Claim 9, wherein said probe pair comprises a split-apart probe pair.

11. The method of Claim 9, wherein said probe pair library comprises: i) a first probe library configured to hybridize to a first region of chromosome nine that is centromeric with respect to the ABL gene, and ii) a second probe library configured to hybridize to a second region of chromosome nine that is teleomeric with respect to the ABL gene.

12. The method of Claim 9, wherein said probe pair library comprises: i) a first probe library configured to hybridize to a first region of chromosome eighteen that is centromeric with respect to the SYT gene, and ii) a second probe library configured to hybridize to a second region of chromosome eighteen that is teleomeric with respect to the SYT gene.

13. The method of any of claims 1 to 12**,** wherein said temperature is at least 98 degrees Celsius.

14. The method of any of claims 1 to 12, wherein said temperature is from 96 degrees Celsius to 100 degrees Celsius.

15. The method of any of claims 1 to 14, wherein said subtracted probe library is about 90 percent free of repeat sequences.

16. The method of any of claims 1 to 14, wherein said subtracted probe library is about 95 percent free of repeat sequences.

17. A method for identifying whether a subject is suitable for treatment with anti-HERZ antibodies,
comprising:
a) preheating a biological sample from a subject in a pretreatment buffer at a temperature of at least 96° Celsius,
b) exposing said biological sample to an enzyme digestion solution,
c) contacting said biological sample with a subtracted probe library under conditions such that said subtracted probe library hybridizes to a target region in said biological sample, wherein said target region comprises the HER2 gene sequence,
d) adding a detection molecule linked to an enzyme to said biological sample under conditions such that said detection molecule binds: i) to said labeled subtracted probe library, or ii) an intermediate molecule linked to said subtracted probe library,
e) adding a colorimetric substrate to said biological sample,
f) detecting said target region by visualizing said colorimetric substrate with a bright-field microscope, thereby determining that said biological sample has amplification of said HER2 gene sequence, and
g) identifying said subject as suitable for treatment with anti-HER2 antibodies.

18. The method of Claim 17, wherein said anti-HER2 antibodies comprise HERCEPTIN^{®}.

## Patentansprüche

1. Ein Verfahren für das Durchführen einer chromogenen in situ-Hybridisierung, das folgendes umfasst:
a) Vorerwärmen einer biologischen Probe in einem Vorbehandlungspuffer bei einer Temperatur von wenigstens 96 Grad Celsius,
b) Aussetzen der biologischen Probe gegenüber einer Enzymverdaulösung,
c) Kontaktieren der biologischen Probe mit einer Subtraktions-Sondenbibliothek unter Bedingungen, die bewirken, dass die Subtraktions-Sondenbibliothek mit einem Zielbereich in der biologischen Probe hybridisiert,
d) Hinzufügen eines an ein Enzym gebundenen Detektionsmoleküls zu der biologischen Probe unter Bedingungen, die bewirken, dass das Detektionsmolekül mit Folgendem bindet: i) der markierten Subtraktions-Sondenbibliothek, oder ii) einem mit der Subtraktions-Sondenbibliothek verbundenen Zwischenmolekül; und
e) Hinzufügen eines kolorimetrischen Substrats zu der biologischen Probe.

2. Das Verfahren gemäß Anspruch 1, das ferner den Schritt f) Detektieren des Zielbereichs umfasst.

3. Das Verfahren gemäß Anspruch 2, wobei das Detektieren das Visualisieren des kolorimetrischen Substrats mit einem Mikroskop umfasst.

4. Das Verfahren gemäß Anspruch 3, wobei das Mikroskop ein Hellfeldmikroskop ist.

5. Das Verfahren gemäß einem der Ansprüche 1 bis 4, wobei die Subtraktions-Sondenbibliothek derart ausgestaltet ist, dass sie eine HER2-Genamplifikation detektiert.

6. Das Verfahren gemäß einem der Ansprüche 1 bis 4, wobei die Subtraktions-Sondenbibliothek derart ausgestaltet ist, dass sie eine TopoIIα-Genamplifikation detektiert.

7. Das Verfahren gemäß einem der Ansprüche 1 bis 4, wobei die Subtraktions-Sondenbibliothek derart ausgestaltet ist, dass sie eine EGFR-Genamplifikation detektiert.

8. Das Verfahren gemäß einem der Ansprüche 1 bis 4, wobei die Subtraktions-Sondenbibliothek derart ausgestaltet ist, dass sie eine N-MYC-Genamplifikation detektiert.

9. Das Verfahren gemäß einem der vorhergehenden Ansprüche, wobei die Subtraktions-Sondenbibliothek eine Sondenpaarbibliothek umfasst.

10. Das Verfahren gemäß Anspruch 9, wobei das Sondenpaar ein getrenntes Sondenpaar umfasst.

11. Das Verfahren gemäß Anspruch 9, wobei die Sondenpaarbibliothek folgendes umfasst:
i) eine erste Sondenbibliothek, die derart ausgestaltet ist, dass sie mit einem ersten Bereich auf Chromosom neun hybridisiert, der zentromerisch zu dem ABL-Gen liegt, und ii) eine zweite Sondenbibliothek, die derart ausgestaltet ist, dass sie mit einem zweiten Bereich von Chromosom neun hybridisiert, der telomerisch zu dem ABL-Gen liegt.

12. Das Verfahren gemäß Anspruch 9, wobei die Sondenpaarbibliothek folgendes umfasst:
i) eine erste Sondenbibliothek, die derart ausgestaltet ist, dass sie mit einem ersten Bereich auf Chromosom achtzehn hybridisiert, der zentromerisch zum SYT-Gen ist, und ii) eine zweite Sondenbibliothek, die derart ausgestaltet ist, dass sie mit einem zweiten Bereich von Chromosom achtzehn hybridisiert, der telomerisch zum SYT-Gen ist.

13. Das Verfahren gemäß einem der Ansprüche 1 bis 12, wobei die Temperatur wenigstens 98 Grad Celsius beträgt.

14. Das Verfahren gemäß einem der Ansprüche 1 bis 12, wobei die Temperatur zwischen 96 Grad Celsius und 100 Grad Celsius liegt.

15. Das Verfahren gemäß einem der Ansprüche 1 bis 14, wobei die Subtraktions-Sondenbibliothek zu etwa 90 Prozent frei von repetitiven Sequenzen ist.

16. Das Verfahren gemäß einem der Ansprüche 1 bis 14, wobei die Subtraktions-Sondenbibliothek zu etwa 95 Prozent frei von repetitiven Sequenzen ist.

17. Ein Verfahren, um zu bestimmen, ob ein Subjekt geeignet für eine Behandlung mit anti-HER2-Antikörpern ist, wobei das Verfahren folgendes umfasst:
a) Vorerwärmen einer biologischen Probe aus einem Subjekt in einem Vorbehandlungspuffer bei einer Temperatur von wenigstens 96 °C,
b) Aussetzen der biologischen Probe gegenüber einer Enzymverdaulösung,
c) Kontaktieren der biologischen Probe mit einer Subtraktions-Sondenbibliothek unter Bedingungen, die bewirken, dass die Subtraktions-Sondenbibliothek mit einem Zielbereich in der biologischen Probe hybridisiert, wobei der Zielbereich die HER2-Gensequenz umfasst,
d) Hinzufügen eines an ein Enzym gebundenen Detektionsmoleküls zu der biologischen Probe unter Bedingungen, die bewirken, dass das Detektionsmolekül mit folgendem bindet: i) der markierten Subtraktions-Sondenbibliothek, oder ii) einem mit der Subtraktions-Sondenbibliothek verbundenen Zwischenmolekül;
e) Hinzufügen eines kolorimetrischen Substrats zu der biologischen Probe,
f) Detektieren des Zielbereichs durch Visualisieren des kolorimetrischen Substrats mit einem Hellfeldmikroskop, wodurch bestimmt wird, dass die biologische Probe eine Amplifikation der HER2-Gensequenz aufweist, und
g) Identifizieren des Subjekts als geeignet für eine Behandlung mit anti-HER2-Antikörpern.

18. Das Verfahren gemäß Anspruch 17, wobei die anti-HER2-Antikörper HERCEPTIN® umfassen.

## Revendications

1. - Procédé pour réaliser une hybridation in situ chromogène, comprenant les opérations consistant à :
a) préchauffer un échantillon biologique dans un tampon de pré-traitement à une température d'au moins 96 degrés Celsius ;
b) exposer ledit échantillon biologique à une solution de digestion enzymatique ;
c) mettre en contact ledit échantillon biologique avec une bibliothèque de sondes soustraites dans des conditions telles que ladite bibliothèque de sondes soustraites s'hybride à une région cible dans ledit échantillon biologique ;
d) ajouter une molécule de détection liée à une enzyme audit échantillon biologique dans des conditions telles que ladite molécule de détection se lie : i) à ladite bibliothèque de sondes soustraites marquées, ou ii) à une molécule intermédiaire liée à ladite bibliothèque de sondes soustraites ; et
e) ajouter un substrat colorimétrique audit échantillon biologique.

2. - Procédé selon la revendication 1, comprenant en outre l'étape f) consistant à détecter ladite région cible.

3. - Procédé selon la revendication 2, dans lequel ladite détection comprend la visualisation dudit substrat colorimétrique avec un microscope.

4. - Procédé selon la revendication 3, dans lequel ledit microscope est un microscope à champ clair.

5. - Procédé selon l'une quelconque des revendications 1 à 4, dans lequel ladite bibliothèque de sondes soustraites est configurée pour détecter l'amplification du gène HER2.

6. - Procédé selon l'une quelconque des revendications 1 à 4, dans lequel ladite bibliothèque de sondes soustraites est configurée pour détecter l'amplification du gène topoIIα.

7. - Procédé selon l'une quelconque des revendications 1 à 4, dans lequel ladite bibliothèque de sondes soustraites est configurée pour détecter l'amplification du gène EGFR.

8. - Procédé selon l'une quelconque des revendications 1 à 4, dans lequel ladite bibliothèque de sondes soustraites est configurée pour détecter l'amplification du gène N-MYC.

9. - Procédé selon l'une quelconque des revendications précédentes, dans lequel ladite bibliothèque de sondes soustraites comprend une bibliothèque de paires de sondes.

10. - Procédé selon la revendication 9, dans lequel ladite paire de sondes comprend une paire de sondes clivées.

11. - Procédé selon la revendication 9, dans lequel ladite bibliothèque de paires de sondes comprend : i) une première bibliothèque de sondes configurée pour s'hybrider à une première région du chromosome neuf qui est centromérique par rapport au gène ABL, et ii) une seconde bibliothèque de sondes configurée pour s'hybrider à une seconde région du chromosome neuf qui est télomérique par rapport au gène ABL.

12. - Procédé selon la revendication 9, dans lequel ladite bibliothèque de paires de sondes comprend : i) une première bibliothèque de sondes configurée pour s'hybrider à une première région du chromosome dix-huit qui est centromérique par rapport au gène SYT, et ii) une seconde bibliothèque de sondes configurée pour s'hybrider à une seconde région du chromosome dix-huit qui est télomérique par rapport au gène SYT.

13. - Procédé selon l'une quelconque des revendications 1 à 12, dans lequel ladite température est d'au moins 98 degrés Celsius.

14. - Procédé selon l'une quelconque des revendications 1 à 12, dans lequel ladite température est de 96 degrés Celsius à 100 degrés Celsius.

15. - Procédé selon l'une quelconque des revendications 1 à 14, dans lequel ladite bibliothèque de sondes soustraites est d'environ 90 pour cent exempte de séquences de répétition.

16. - Procédé selon l'une quelconque des revendications 1 à 14, dans lequel ladite bibliothèque de sondes soustraites est d'environ 95 pour cent exempte de séquences de répétition.

17. - Procédé pour identifier si un sujet est approprié pour un traitement avec des anticorps anti-HER2, comprenant les opérations consistant à :
a) préchauffer un échantillon biologique provenant d'un sujet dans un tampon de prétraitement à une température d'au moins 96° Celsius ;
b) exposer ledit échantillon biologique à une solution de digestion enzymatique ;
c) mettre en contact ledit échantillon biologique avec une bibliothèque de sondes soustraites dans des conditions telles que ladite bibliothèque de sondes soustraites s'hybride à une région cible dans ledit échantillon biologique, ladite région cible comprenant la séquence du gène HER2 ;
d) ajouter une molécule de détection liée à une enzyme audit échantillon biologique dans des conditions telles que ladite molécule de détection se lie : i) à ladite bibliothèque de sondes soustraites marquées, ou ii) à une molécule intermédiaire liée à ladite bibliothèque de sondes soustraites ;
e) ajouter un substrat colorimétrique audit échantillon biologique ;
f) détecter ladite région cible en visualisant ledit substrat colorimétrique avec un microscope à champ clair, déterminant ainsi que ledit échantillon biologique a une amplification de ladite séquence du gène HER2 ; et
g) identifier ledit sujet comme étant approprié pour un traitement avec des anticorps anti-HER2.

18. - Procédé selon la revendication 17, dans lequel lesdits anticorps anti-HER2 comprennent l'HERCEPTIN®.


### SEQUENCE LISTING

<110> Zymed Laboratories, Inc.
<120> Chromogenic In Situ Hybridization Methods, Kits and Compositions
<130> ZYLA021PEP
<140> 02 761 652.3
   <141> 2002-09-13
<150> PCT/US02/29205
   <151> 2002-09-13
<150> 09/952,851
   <151> 2001-09-14
<150> 10/173,525
   <151> 2002-06-17
<160> 31
<170> PatentIn version 3.1
<210> 1
   <211> 22
   <212> DNA
   <213> Artificial Sequence
<220>
   <223> Synthetic
<400> 1
   gcctccctaa cctgattggt tt 22
<210> 2
   <211> 22
   <212> DNA
   <213> Artificial Sequence
<220>
   <223> Synthetic
<400> 2
   ctgaagaacc ctgaaagcga ct 22
<210> 3
   <211> 21
   <212> DNA
   <213> Artificial Sequence
<220>
   <223> Synthetic
<400> 3
   ctggctccga tgtatttgat g 21
<210> 4
   <211> 22
   <212> DNA
   <213> Artificial Sequence
<220>
   <223> Synthetic
<400> 4
   cctgcccata agtctctctg ct 22
<210> 5
   <211> 20
   <212> DNA
   <213> Artificial Sequence
<220>
   <223> Synthetic
<400> 5
   gattagcctg ccctctttgg 20
<210> 6
   <211> 20
   <212> DNA
   <213> Artificial Sequence
<220>
   <223> Synthetic
<400> 6
   cagaagggag gcagacagtc 20
<210> 7
   <211> 21
   <212> DNA
   <213> Artificial Sequence
<220>
   <223> Synthetic
<400> 7
   gctcatggtg tcaggaggat g 21
<210> 8
   <211> 21
   <212> DNA
   <213> Artificial Sequence
<220>
   <223> Synthetic
<400> 8
   gcaggaatag gtgggatgga g 21
<210> 9
   <211> 406
   <212> DNA
   <213> Artificial Sequence
<220>
   <223> Synthetic
<220>
   <221> misc_feature
   <222> (16)..(16)
   <223> The n at this position can be a, c, t, or g.
<400> 9
   gaagatacat ccaaantcca gcctacgcaa cagagcagga ttcagtctca aaaaaagaaa 60
   aaaagaaaag aaaacgttcc ccaccccatc tccttccttg atcatcactg gaccctgttc 120
   tgccaccaac ttgcgtgaac ttggagtttg actgacctta gctgtaacat ggaggtagat 180
   catctccacc catcctacct cttgaagctc ttgtgagagt aaaatgaatg gagaagagta 240
   gttctgctcc caatgccaga catgtgccct gttcagcaag cccaagagga gaaaaggtgc 300
   caggacacag aggcaggagt gcaggagagg ccggacaaac ccacgcaaca tgcctgggat 360
   gaagcatgag tgcaggtgag tgtgggaatc tgcaaaggtt gccaga 406
<210> 10
   <211> 750
   <212> DNA
   <213> Artificial Sequence
<220>
   <223> Synthetic
<220>
   <221> misc_feature
   <222> (3)..(3)
   <223> The n at this position can be a, c, t, or g.
<220>
   <221> misc_feature
   <222> (5)..(6)
   <223> The n at this position can be a, c, t, or g.
<220>
   <221> misc_feature
   <222> (8)..(8)
   <223> The n at this position can be a, c, t, or g.
<220>
   <221> misc_feature
   <222> (16)..(16)
   <223> The n at this position can be a, c, t, or g.
<220>
   <221> misc_feature
   <222> (27)..(27)
   <223> The n at this position can be a, c, t, or g.
<220>
   <221> misc_feature
   <222> (34)..(35)
   <223> The n at this position can be a, c, t, or g.
<220>
   <221> misc_feature
   <222> (52)..(52)
   <223> The n at this position can be a, c, t, or g.
<220>
   <221> misc_feature
   <222> (54)..(54)
   <223> The n at this position can be a, c, t, or g.
<220>
   <221> misc_feature
   <222> (64)..(65)
   <223> The n at this position can be a, c, t, or g.
<220>
   <221> misc_feature
   <222> (85)..(85)
   <223> The n at this position can be a, c, t, or g.
<220>
   <221> mise_feature
   <222> (87)..(87)
   <223> The n at this position can be a, c, t, or g.
<220>
   <221> misc_feature
   <222> (96)..(96)
   <223> The n at this position can be a, c, t, or g.
<220>
   <221> misc_feature
   <222> (108)..(108)
   <223> The n at this position can be a, c, t, or g.
<220>
   <221> misc_feature
   <222> (112)..(112)
   <223> The n at this position can be a, c, t, or g.
<220>
   <221> mise_feature
   <222> (122)..(122)
   <223> The n at this position can be a, c, t, or g.
<220>
   <221> mise_feature
   <222> (131)..(131)
   <223> The n at this position can be a, c, t, or g.
<220>
   <221> misc_feature
   <222> (133)..(133)
   <223> The n at this position can be a, c, t, or g.
<220>
   <221> misc_feature
   <222> (163)..(163)
   <223> The n at this position can be a, c, t, or g.
<220>
   <221> misc_feature
   <222> (165)..(165)
   <223> The n at this position can be a, c, t, or g.
<220>
   <221> misc_feature
   <222> (178)..(178)
   <223> The n at this position can be a, c, t, or g.
<220>
   <221> misc_feature
   <222> (181)..(182)
   <223> The n at this position can be a, c, t, or g.
<220>
   <221> misc_feature
   <222> (199)..(199)
   <223> The n at this position can be a, c, t, or g.
<220>
   <221> misc-feature
   <222> (210)..(210)
   <223> The n at this position can be a, c, t, or g.
<220>
   <221> misc_feature
   <222> (218)..(218)
   <223> The n at this position can be a, c, t, or g.
<220>
   <221> misc_feature
   <222> (231)..(231)
   <223> The n at this position can be a, c, t, or g.
<220>
   <221> misc_feature
   <222> (287)..(287)
   <223> The n at this position can be a, c, t, or g.
<220>
   <221> misc-feature
   <222> (297)..(297)
   <223> The n at this position can be a, c, t, or g.
<220>
   <221> misc_feature
   <222> (312)..(312)
   <223> The n at this position can be a, c, t, or g.
<220>
   <221> misc_feature
   <222> (321)..(321)
   <223> The n at this position can be a, c, t, or g.
<220>
   <221> misc_feature
   <222> (323)..(323)
   <223> The n at this position can be a, c, t, or g.
<220>
   <221> misc_feature
   <222> (334)..(334)
   <223> The n at this position can be a, c, t, or g.
<220>
   <221> misc_feature
   <222> (338)..(338)
   <223> The n at this position can be a, c, t, or g.
<220>
   <221> misc_feature
   <222> (368)..(368)
   <223> The n at this position can be a, c, t, or g.
<220>
   <221> misc_feature
   <222> (414)..(414)
   <223> The n at this position can be a, c, t, or g.
<220>
   <221> misc_feature
   <222> (416)..(416)
   <223> The n at this position can be a, c, t, or g.
<220>
   <221> misc_feature
   <222> (434)..(434)
   <223> The n at this position can be a, c, t, or g.
<220>
   <221> misc_feature
   <222> (436)..(436)
   <223> The n at this position can be a, c, t, or g.
<220>
   <221> misc_feature
   <222> (443)..(443)
   <223> The n at this position can be a, c, t, or g.
<220>
   <221> mise_feature
   <222> (447)..(447)
   <223> The n at this position can be a, c, t, or g.
<220>
   <221> misc_feature
   <222> (451)..(451)
   <223> The n at this position can be a, c, t, or g.
<220>
   <221> misc_feature
   <222> (461)..(461)
   <223> The n at this position can be a, c, t, or g.
<220>
   <221> misc_feature
   <222> (467)..(467)
   <223> The n at this position can be a, c, t, or g.
<220>
   <221> misc_feature
   <222> (479)..(479)
   <223> The n at this position can be a, c, t, or g.
<220>
   <221> misc_feature
   <222> (481)..(481)
   <223> The n at this position can be a, c, t, or g.
<220>
   <221> misc-feature
   <222> (489) .. (489)
   <223> The n at this position can be a, c, t, or g.
<220>
   <221> misc_feature
   <222> (508)..(508)
   <223> The n at this position can be a, c, t, or g.
<220>
   <221> misc_feature
   <222> (530)..(530)
   <223> The n at this position can be a, c, t, or g.
<220>
   <221> misc_feature
   <222> (533)..(533)
   <223> The n at this position can be a, c, t, or g.
<220>
   <221> misc_feature
   <222> (554)..(554)
   <223> The n at this position can be a, c, t, or g.
<220>
   <221> mise_feature
   <222> (557)..(558)
   <223> The n at this position can be a, c, t, or g.
<220>
   <221> misc_feature
   <222> (574)..(574)
   <223> The n at this position can be a, c, t, or g.
<220>
   <221> misc_feature
   <222> (578)..(578)
   <223> The n at this position can be a, c, t, or g.
<220>
   <221> misc-feature
   <222> (585)..(585)
   <223> The n at this position can be a, c, t, or g.
<220>
   <221> misc_feature
   <222> (587)..(587)
   <223> The n at this position can be a, c, t, or g.
<220>
   <221> misc_feature
   <222> (590)..(590)
   <223> The n at this position can be a, c, t, or g.
<220>
   <221> misc_feature
   <222> (596)..(596)
   <223> The n at this position can be a, c, t, or g.
<220>
   <221> misc_feature
   <222> (600)..(600)
   <223> The n at this position can be a, c, t, or g.
<220>
   <221> misc_feature
   <222> (602)..(602)
   <223> The n at this position can be a, c, t, or g.
<220>
   <221> mise_feature
   <222> (605)..(605)
   <223> The n at this position can be a, c, t, or g.
<220>
   <221> misc_feature
   <222> (607)..(607)
   <223> The n at this position can be a, c, t, or g.
<220>
   <221> mise_feature
   <222> (619)..(622)
   <223> The n at this position can be a, c, t, or g.
<220>
   <221> mise_feature
   <222> (632)..(632)
   <223> The n at this position can be a, c, t, or g.
<220>
   <221> misc_feature
   <222> (646)..(646)
   <223> The n at this position can be a, c, t, or g.
<220>
   <221> misc_feature
   <222> (649)..(649)
   <223> The n at this position can be a, c, t, or g.
<220>
   <221> misc_feature
   <222> (653)..(653)
   <223> The n at this position can be a, c, t, or g.
<220>
   <221> misc_feature
   <222> (658)..(658)
   <223> The n at this position can be a, c, t, or g.
<220>
   <221> misc_feature
   <222> (663)..(663)
   <223> The n at this position can be a, c, t, or g.
<220>
   <221> misc_feature
   <222> (666)..(666)
   <223> The n at this position can be a, c, t, or g.
<220>
   <221> misc_feature
   <222> (669)..(669)
   <223> The n at this position can be a, c, t, or g.
<220>
   <221> misc_feature
   <222> (677)..(677)
   <223> The n at this position can be a, c, t, or g.
<220>
   <221> misc_feature
   <222> (682)..(682)
   <223> The n at this position can be a, c, t, or g.
<220>
   <221> misc_feature
   <222> (690)..(690)
   <223> The n at this position can be a, c, t, or g.
<220>
   <221> misc_feature
   <222> (711)..(711)
   <223> The n at this position can be a, c, t, or g.
<220>
   <221> misc_feature
   <222> (715)..(715)
   <223> The n at this position can be a, c, t, or g.
<220>
   <221> misc_feature
   <222> (718)..(718)
   <223> The n at this position can be a, c, t, or g.
<220>
   <221> misc_feature
   <222> (721)..(721)
   <223> The n at this position can be a, c, t, or g.
<220>
   <221> mise feature
   <222> (724)..(724)
   <223> The n at this position can be a, c, t, or g.
<220>
   <221> misc_feature
   <222> (728)..(728)
   <223> The n at this position can be a, c, t, or g.
<220>
   <221> mise feature
   <222> (731)..(731)
   <223> The n at this position can be a, c, t, or g.
<220>
   <221> misc_feature
   <222> (739)..(739)
   <223> The n at this position can be a, c, t, or g.
<220>
   <221> misc_feature
   <222> (743)..(743)
   <223> The n at this position can be a, c, t, or g.
<220>
   <221> mise feature
   <222> (745)..(745)
   <223> The n at this position can be a, c, t, or g.
<400> 10
   gcngnngnca agcctnccaa ggtaggnttc cgannggcgg ccgcctggcc gncnacattt     60
   aagnngacac tatagaagga tcgtngnatt gttgcntccc tctttacngg cnctatggct    120
   cnattttgtt ngntactgag gggtaaaaga taaatgttta ccntnaccta aaattggntt    180
   nnggcctcta aaggaaccng aggcttaaan gaattatngg ctttggaagc nggccttcaa    240
   attactgcgc taatttatat ttttcattaa aaactcagct ggcctcntct atatagntgt    300
   cttccctggc cntgaaaccc nantgtttcg ccanaaanga ttttaaaatt aaggggtcat    360
   aattcccncc ccatgatgtg tggattaatg gtaagaagga tgcccagaac gttntnttct    420
   taggttgaac gaananaaaa gtnaaanagt ngggctctgg nttctcncct ttgaagccnc    480
   ncaattcgng agatactatg ctgaaccnta gtttttcttt atataggggn gtngaacttt    540
   accctcaaaa tcantanntc agcacatcaa gganattntg gatcntnggn tcttcnctgn    600
   cnccnanatg ctgggaccnn nnaccttgca tnaacagttt gctttngtnc ctntgcanag 660
   ggntgngcnt ttccaanagg gnaaggcaan ggcctaacat catacctggg ngccnagnaa 720
   nccnaaanac ngggaaggnc tcncntaccc 750
<210> 11
   <211> 22
   <212> DNA
   <213> Artificial Sequence
<220>
   <223> Synthetic
<400> 11
   gcctccctaa cctgattggt ta 22
<210> 12
   <211> 22
   <212> DNA
   <213> Artificial Sequence
<220>
   <223> Synthetic
<400> 12
   ctcaagaacc ctgaaagcga ct 22
<210> 13
   <211> 23
   <212> DNA
   <213> Artificial Sequence
<220>
   <223> Synthetic
<400> 13
   gctacggtct gctcaggaca gtt 23
<210> 14
   <211> 21
   <212> DNA
   <213> Artificial Sequence
<220>
   <223> Synthetic
<400> 14
   ctgtcctgag cataccgtag c 21
<210> 15
   <211> 22
   <212> DNA
   <213> Artificial Sequence
<220>
   <223> Synthetic
<400> 15
   aattcttgcg ccttaaacca ac 22
<210> 16
   <211> 18
   <212> DNA
   <213> Artificial Sequence
<220>
   <223> Synthetic
<400> 16
   gttggtttaa ggcgcaag 18
<210> 17
   <211> 22
   <212> DNA
   <213> Artificial Sequence
<220>
   <223> Synthetic
<400> 17
   aattcttgcg ccttaaacca ac 22
<210> 18
   <211> 21
   <212> DNA
   <213> Artificial Sequence
<220>
   <223> Synthetic
<400> 18
   ctgagcggaa ttcgtgagac c 21
<210> 19
   <211> 23
   <212> DNA
   <213> Artificial Sequence
<220>
   <223> Synthetic
<400> 19
   ggtctcacga attccgctca gtt 23
<210> 20
   <211> 23
   <212> DNA
   <213> Artificial Sequence
<220>
   <223> Synthetic
<400> 20
   accgtaggac tctgctggcg att 23
<210> 21
   <211> 21
   <212> DNA
   <213> Artificial Sequence
<220>
   <223> Synthetic
<400> 21
   tcgccagcag agtcctacgg t 21
<210> 22
   <211> 23
   <212> DNA
   <213> Artificial Sequence
<220>
   <223> Synthetic
<400> 22
   atcggtgtag cctgaatgga ctt 23
<210> 23
   <211> 21
   <212> DNA
   <213> Artificial Sequence
<220>
   <223> Synthetic
<400> 23
   gtccattcag gctacaccga t 21
<210> 24
   <211> 23
   <212> DNA
   <213> Artificial Sequence
<220>
   <223> Synthetic
<400> 24
   catcattcgg tcagaggcac ttt 23
<210> 25
   <211> 21
   <212> DNA
   <213> Artificial Sequence
<220>
   <223> Synthetic
<400> 25
   agtgcctctg accgaatgat g 21
<210> 26
   <211> 21
   <212> DNA
   <213> Artificial Sequence
<220>
   <223> Synthetic
<400> 26
   tcttcacgac acgacagcca g 21
<210> 27
   <211> 23
   <212> DNA
   <213> Artificial Sequence
<220>
   <223> Synthetic
<400> 27
   ctggctgtcg tgtcgtgaag att 23
<210> 28
   <211> 21
   <212> DNA
   <213> Artificial Sequence
<220>
   <223> Synthetic
<400> 28
   atgcgtccac cttgacctta c 21
<210> 29
   <211> 23
   <212> DNA
   <213> Artificial Sequence
<220>
   <223> Synthetic
<400> 29
   gtaaggtcaa ggtggacgca ttt 23
<210> 30
   <211> 21
   <212> DNA
   <213> Artificial Sequence
<220>
   <223> Synthetic
<400> 30
   atagccccga atcaggtgga a 21
<210> 31
   <211> 23
   <212> DNA
   <213> Artificial Sequence
<220>
   <223> Synthetic
<400> 31
   ttccacctga ttcggggcta ttt 23